# EUROPEAN PATENT APPLICATION

(11) **EP 4 692 060 A2**
(43) Date of publication of application: **11.02.2026**
(21) Application number: 25215683.1
(22) Date of filing: 16.12.2022
(51) Int. Cl.: C07D 211/42

(54) **METHOD FOR PREPARATION OF (2R, 3S)-2-(BENZO[D]IMIDAZOLYLPROPYL)PIPERIDIN-3-OL DERIVATIVES**

(30) Priority: 17.12.2021 KR 20210182187
(62) Divisional of application: 22907999.1
(71) Applicant: Daewoong Pharmaceutical Co., Ltd., Hwaseong-si, Gyenoggi-do 18623 (KR)
(72) Inventor: LEE, Joon-Hwan, Seoul 06170 (KR); CHO, Min Jae, Seoul 06170 (KR); PARK, Joon Seok, Seoul 06170 (KR)
(74) Representative: Germain Maureau

(57) **Abstract**

The present invention relates to a method for preparing (2R, 3S)-2-(benzo[d]imidazolylpropyl)piperidin-3-ol derivatives, and the preparation method according to the present invention has the advantage that the compound can be prepared in high yield even with a shortened process.

## Description

### [TECHNICAL FIELD]

The present invention relates to a method for preparing (2R,3S)-2-(benzo[d]imidazolylpropyl)piperidin-3-ol derivatives.

### [BACKGROUND]

PRS (prolyl-tRNA synthetase) is one of the aminoacyl-tRNA synthetase (ARS) family and serves to activate an amino acid for protein synthesis. That is, ARS performs a translational function to form aminoacyl adenylate (AA-AMP) and then transfer the activated amino acid to the 3-end of the corresponding tRNA. Since ARS plays an important role in the synthesis of protein, ARS inhibitors suppress the growth of all cells. Thus, ARS has been recognized as a promising target for a therapeutic agent for treating diseases that should suppress antibiotics or cell overexpression (Nature, 2013, 494:121-125).

PRS is present in, or functions as, a multi-synthetase complex (MSC) in the form of EPRS (Glutamyl-Prolyl-tRNA Synthetase). In particular, among various MSCs, EPRS functions as a translational silencer that suppresses the production of VEGF A (vascular endothelial growth factor A) which is a key factor in angiogenesis. In addition, it is reported that EPRS is closely related with various solid tumors (Nat. Rev. Cancer, 2011, 11, 708-718).

The only substance, known as the PRS inhibitor, is halofuginone. Halofuginone is a derivative of febrifugine derived from natural products and has anti-malarial effects and various anti-inflammatory effects. It can also be used as an animal feed additive. In addition, it has been reported that halofuginone increases the phosphorylation of GCN2 kinase through PRS inhibition, which induces ATF4 and CHOP expression, and thus promotes cell death (Nat. Chem. Biol. 2012, 8, 311-317). Currently, halofuginone is being clinically studied as anti-cancer agent, an anti-inflammatory agent (J Immunol, 2014, 192(5), 2167-76), therapeutic agents for the treatment of autoimmune diseases (Arthritis Rheumatol, 2014, 66 (5), 1195-207), therapeutic agents for the treatment of fibrosis diseases (World J Gastroenterol, 2014, 20 (40), 14778-14786), and the like (Bioorg. Med. Chem. 2014, 22, 1993-2004).

However, it has been reported that halofuginone acts on various targets and has a very severe toxicity and further there is a risk of genotoxicity (The EFSA Journal, 2003, 8: 1-45). Therefore, discovering PRS inhibitors having higher safety to the human body among substances capable of inhibiting PRS like halofuginone has a significance in terms of developing an anti-cancer agent of the next generation that can be used as an antifibrosis agent, an anti-inflammatory agent, an autoimmune therapeutic agent alone or in combination with an existing targeted anti-cancer agent.

In this regard, the present inventors have confirmed that (2R, 3S)-2-(benzo[d]imidazolylpropyl)piperidin-3-ol derivatives, which have a different chemical structure from PRS enzyme inhibitors reported to date, can exhibit excellent PRS activity inhibitory effect. Thus, the present inventors have conducted intensive studies to develop a preparation method that can commercially prepare (2R, 3S)-2-(benzo[d]imidazolylpropyl)piperidin-3-ol derivatives, and found that when the preparation method described hereinafter is used, commercial mass production is possible, and overall yield is improved, thereby completing the present invention.

### [DETAILED DESCRIPTION OF THE INVENTION]

### [Technical Problem]

It is an object of the present invention to provide a method for preparing a compound represented by Chemical Formula 1 described hereinafter, i.e., (2R,3S)-2-(3-(4,5-dichloro-1H-benzo[d]imidazol-1-yl)propyl)piperidin-3-ol monohydrochloride, which is (2R,3S)-2-(benzo[d]imidazolylpropyl)piperidin-3-ol derivative.

It is another object of the present invention to provide a method for preparing a compound represented by Chemical Formula 1-1 described hereinafter, which is a starting material in the method for preparing the (2R,3S)-2-(benzo[d]imidazolylpropyl)piperidin-3-ol derivative.

It is yet another object of the present invention to provide a method for preparing a compound represented by Chemical Formula 2 described hereinafter, which is an intermediate compound for the preparation of a compound represented by Chemical Formula 1-1 described hereinafter.

### [Technical Solution]

In order to achieve the above objects, provided herein is a method for preparing (2R,3S)-2-(3-(4,5-dichloro-1H-benzo[d]imidazol-1-yl)propyl)piperidin-3-ol 1HCl, which is a compound represented by the following Chemical Formula 1, and more specifically, provided is a preparation method comprising the steps of:
1) reacting a compound represented by the following Chemical Formula 1-1 with a compound represented by the following Chemical Formula 1-2 in the presence of a base to prepare a compound represented by the following Chemical Formula 1-3;
2) reacting a compound represented by the following Chemical Formula 1-3 in the presence of a sulfur-containing reducing agent to prepare a compound represented by the following Chemical Formula 1-4;
3) reacting a compound represented by the following Chemical Formula 1-4 with an orthoformate-based compound to prepare a compound represented by the following Chemical Formula 1-5; and
4) reacting a compound represented by the following Chemical Formula 1-5 with hydrochloric acid in the presence of water and acetone to prepare a compound represented by the following Chemical Formula 1: 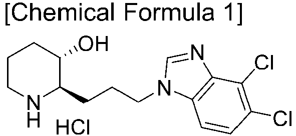 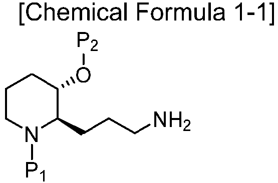 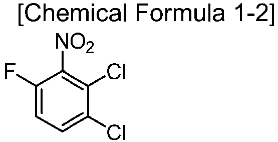 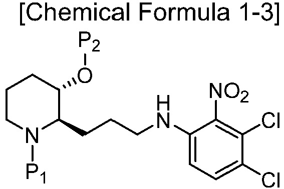 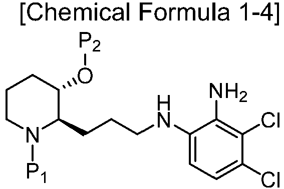 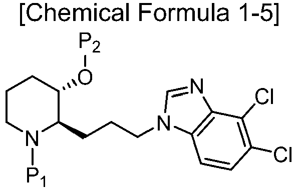
   wherein, in Chemical Formulas 1-1 to 1-5,
   P₁ and P₂ each independently represent a protecting group.

Also provided therein is a method for preparing a compound represented by the following Chemical Formula 1-1, which is a starting material used in the method for preparing the compound represented by the following Chemical Formula 1, and more specifically, provided is a preparation method comprising the steps of:
1) reacting a compound represented by the following Chemical Formula 1A with pyrrolidine to prepare a compound represented by the following Chemical Formula 1B;
2) reacting a compound represented by the following Chemical Formula 1B with 3-bromo-1-propene to prepare a compound represented by the following Chemical Formula 1C;
3) reducing a compound represented by the following Chemical Formula 1C in the presence of a ketoreductase enzyme to prepare a compound represented by the following Chemical Formula 1D;
4) reacting a compound represented by the following Chemical Formula 1D with a compound represented by the following Chemical Formula 1d to prepare a compound represented by the following Chemical Formula 1E;
5) ① reacting a compound represented by the following Chemical Formula 1E with a borane-based compound and then ② reacting the reactant with an oxidizing agent to prepare a compound represented by the following Chemical Formula 1F;
6) reacting a compound represented by the following Chemical Formula 1F with methanesulfonyl chloride in the presence of a base to prepare a compound represented by the following Chemical Formula 1G;
7) reacting a compound represented by the following Chemical Formula 1G with an azidation reagent to prepare a compound represented by the following Chemical Formula 1H; and
8) reacting a compound represented by the following Chemical Formula 1H in the presence of a base to prepare a compound represented by the following Chemical Formula 1-1:

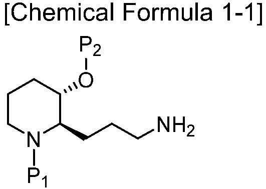

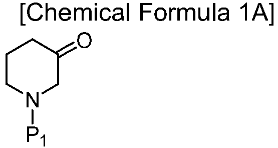

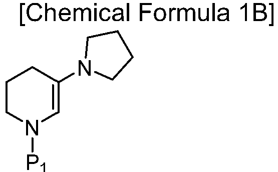

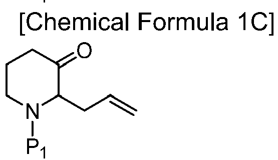

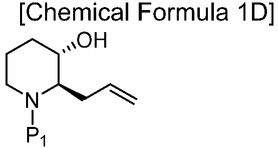

   [Chemical Formula 1d] P₂-X

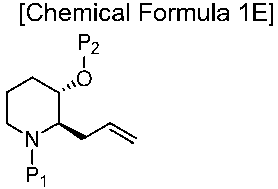

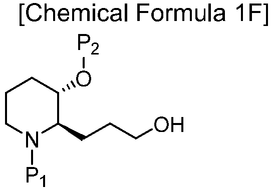

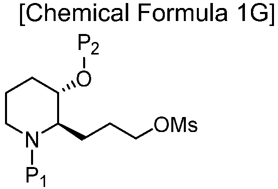

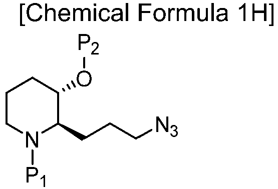

   **wherein,** in Chemical Formulas 1-1, 1A to 1H and 1d,
   X is halogen,
   Ms is methanesulfonyl, and
   P₁ and P₂ each independently represent a protecting group.

Further provided herein is a method for preparing a compound represented by the following Chemical Formula 2, which is an intermediate for the preparation of the compound represented by Chemical Formula 1-1, and more specifically, provided is a preparation method comprising the steps of:
1) reacting a compound represented by the following Chemical Formula 2-1 with propane-1,3-diol to prepare a compound represented by the following Chemical Formula 2-2;
2) oxidizing a compound represented by the following Chemical Formula 2-2 to prepare a compound represented by the following Chemical Formula 2-3;
3) ① reacting a compound represented by the following Chemical Formula 2-3 with dibenzyl azodicarboxylate or di(C₁₋₄ alkyl)azodicarboxylate, and then ② reacting the reactant withallyl halide to prepare a compound represented by the following Chemical Formula 2-4;
4) subjecting a compound represented by the following Chemical Formula 2-4 to a cyclization reaction to prepare a compound represented by the following Chemical Formula 2-5;
5) removing benzyl carbamate or C₁₋₄ alkyl carbamate from a compound represented by the following Chemical Formula 2-5 to prepare a compound represented by the following Chemical Formula 2-6;
6) subjecting a compound represented by the following Chemical Formula 2-6 to a boronation-oxidation reaction to prepare a compound represented by the following Chemical Formula 2-7;
7) subjecting a compound represented by the following Chemical Formula 2-7 to a cyclization reaction to prepare a compound represented by the following Chemical Formula 2-8;
8) reacting a compound represented by the following Chemical Formula 2-8 in the presence of a base to prepare a compound represented by the following Chemical Formula 2-9;
9) introducing a protecting group into a compound represented by the following Chemical Formula 2-9 to prepare a compound represented by the following Chemical Formula 2-10;
10) reacting a compound represented by the following Chemical Formula 2-10 in the presence of an acid to prepare a compound represented by the following Chemical Formula 2-11;
11) introducing a protecting group into a compound represented by the following Chemical Formula 2-11 to prepare a compound represented by the following Chemical Formula 2-12; and
12) reacting a compound represented by the following Chemical Formula 2-12 in the presence of an acid to prepare a compound represented by the following Chemical Formula 2: 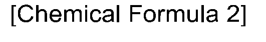 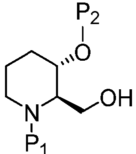 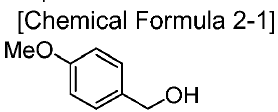 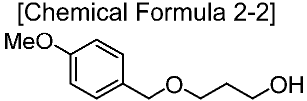 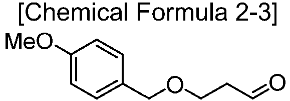 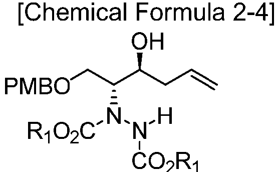 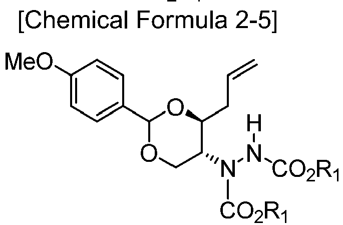 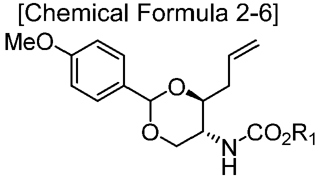 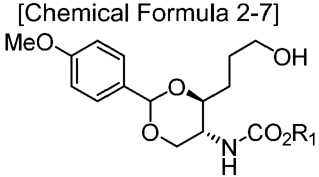 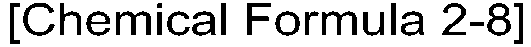 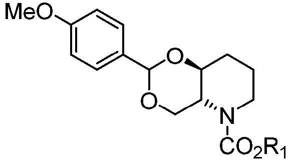 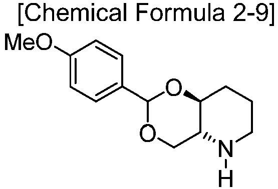 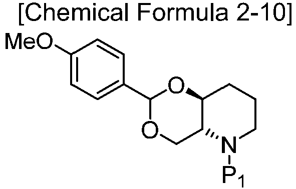 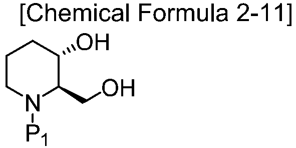 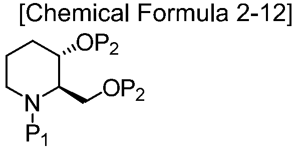
   wherein, in Chemical Formulas 2 and 2-1 to 2-12,
   R₁ is benzyl or C₁₋₄ alkyl, and
   P₁ and P₂ each independently represent a protecting group.

Hereinafter, the preparation method of each compound will be described in detail.

### (Method for preparing the compound represented by Chemical Formula 1)

Meanwhile, the compound represented by Chemical Formula 1 is prepared in accordance with the following Reaction Scheme 1: 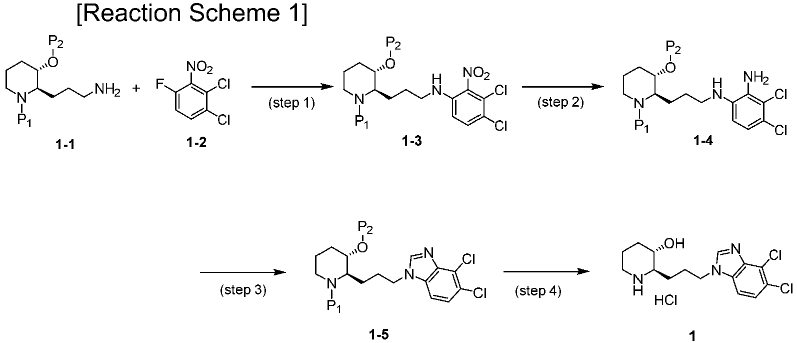 in Reaction Scheme 1, P₁ and P₂ each independently represent a protecting group.

More specifically, P₁ may be any one protecting group selected from the group consisting of tert-butyloxycarbonyl(Boc), carbobenzyloxy(Cbz), paramethoxybenzylcarbonyl(Moz), 9-fluorenylmethyloxycarbonyl(Fmoc), acetyl(Ac), benzoyl(Bz), benzyl(Bn) and para-methoxybenzyl(PMB).

In addition, P₂ may be any one protecting group selected from the group consisting of tert-butyldimethylsilyl(TBS), 2-methoxyethoxymethyl ether(MEM), methoxymethyl ether(MOM), para-methoxybenzyl(PMB) ether, methylthiomethyl ether, pivaloyl (Piv), tetrahydropyranyl(THP), trityl(triphenylmethyl, Tr), tert-butyldimethylsilyl(TBDMS), triisopropylsilyl(TIPS) ether and ethoxyethyl ether(EE).

At this time, it is preferable in terms of ease of preparation and yield that P₁ is tert-butyloxycarbonyl (Boc), and P₂ is tert-butyldimethylsilyl (TBS).

### Step 1)

Step 1 is a step of reacting the compound represented by Chemical Formula 1-1 with the compound represented by Chemical Formula 1-2 in the presence of a base to prepare the compound represented by Chemical Formula 1-3, wherein the reaction is an amine substitution reaction, which is carried out in the presence of a base.

In the above step, the compound represented by Chemical Formula 1-1 and the compound represented by Chemical Formula 1-2 may be used in an equivalent ratio of 1:0.5 to 1:1.5. Specifically, the compound represented by Chemical Formula 1-1 and the compound represented by Chemical Formula 1-2 may be used in an equivalent ratio of 1:0.7 to 1:1.3, 1:0.9 to 1:1.1, or 1:1.

Further, in the above step, the base may be at least one selected from the group consisting of N,N-diisopropylethylamine(DIPEA), diisopropylamine, triethylaminde (TEA), pyridine, potassium carbonate(K₂CO₃) and sodium carbonate(Na₂CO₃). Among these, it is advantageous to use diisopropylethylamine or potassium carbonate as a base in terms of reaction rate and yield. Particularly, it is most preferable to use potassium carbonate.

Further, the base may be used in an amount of 1 to 4 equivalents relative to 1 equivalent of the compound represented by Chemical Formula 1-1. If the base is used in an excessively low amount, there is a possibility that the reaction time becomes longer, and even if the base is used in an excessively large amount, there is no difference in reaction time, and thus, it is preferable to use the base within the above-mentioned range.

And, the reaction may be carried out in at least one organic solvent selected from the group consisting of tetrahydrofuran (THF), 1,4-dioxane, acetonitrile (ACN) and N,N-dimethylformamide (DMF).

When N,N-dimethylformamide (DMF) is used as a solvent in the above reaction, there is a problem that not only the solvent is not completely removed even if a work-up is carried out after completing the reaction, but also the residual amount of solvent is not constant for each batch. Furthermore, if the residual amount of solvent is not constant, the crystallization yield and the reproducibility of the quality of the final compound may vary depending on the residual amount, which is thus not preferable.

However, when tetrahydrofuran (THF) or 1,4-dioxane is used as a solvent for the reaction, there is an advantage that it can be completely removed after the reaction is completed. Tetrahydrofuran (THF) is particularly preferred because it has a fast reaction rate and generates few impurities.

Further, the organic solvent may be used in an amount (mL/g) of 5 to 30 times (volume), more specifically in an amount (mL/g) of 5 to 15 times (volume), relative to the weight of the compound represented by Chemical Formula 1-1.

The reaction may be carried out at the reflux temperature of the organic solvent. Specifically, the reaction may be carried out at a temperature of 60 to 120°C for 3 to 10 hours. When the reaction is carried out under a lower temperature condition and/or for a shorter reaction time than the above-mentioned range, the reaction does not proceed sufficiently, and the production yield may be lowered. In addition, even if the reaction is carried out under a higher temperature condition and/or a longer reaction time than the above-mentioned range, the production yield is not substantially increased, which is not preferable in terms of process cost. As an example, when tetrahydrofuran is used as the organic solvent, the reaction may be carried out at a tetrahydrofuran reflux temperature of 80 to 100°C for 4 hours to 7 hours.

Further, the above step may further comprise a step of crystallizing the reaction product with a solvent in order to purify the compound represented by Chemical Formula 1-3. When the reaction product is crystallized with a solvent in this way, it can be applied to industrialization, unlike the case where the reaction product is purified using a silica column, thereby being advantageous in terms of industrial mass production.

Meanwhile, after the reaction is completed, the method may further comprise a step of crystallizing the reaction product with ethanol and water, if necessary. Preferably, the crystallization consists of two steps, and specifically, is carried out by a primary crystallization with ethanol and a secondary crystallization with purified water, which makes the crystal formation best. When crystallization is performed in two steps in this way, it is possible to prevent a phenomenon in which crystals are formed and adhered to the wall surface, thereby preventing a decrease in yield. Products that have not been crystallized can be further crystallized in the two-step crystallization step.

At this time, ethanol may be used in an amount (mL/g) of 3 to 5 times (volume) relative to the weight of the compound represented by Chemical Formula 1-1. Further, the volume ratio of ethanol and water may be 1:0.5 to 1:1.5, preferably 1:1.

### Step 2)

Step 2 is a step of preparing the compound represented by Chemical Formula 1-4 through a reduction reaction of the compound represented by Chemical Formula 1-3, wherein the reduction reaction is carried out in the presence of a sulfur-containing reducing agent.

The reaction of reducing a nitro group to an amino group as mentioned above has generally been carried out using hydrogen gas in the presence of a catalyst, but there were concerns about safety due to the toxicity of the catalyst and the explosive nature of hydrogen gas. In particular, when reduction is carried out using metal catalysts such as Raney nickel and palladium carbon (Pd/C) and hydrogen gas, it was unsuitable for application as an industrial process due to the highly toxic and fire hazardous nickel and palladium metal catalysts and the highly explosive hydrogen gas.

Specifically, the sulfur-containing reducing agent may be at least one selected from the group consisting of sodium hydrosulfite (sodium dithionite; Na₂S₂O₄), sodium bisulfite, sodium metabisulfite and sodium sulfide, but is not limited thereto.

Further, the sulfur-containing reducing agent may be used in an amount of 1 to 10 equivalents relative to 1 equivalent of the compound represented by Chemical Formula 1-3. When the sulfur-containing reducing agent is used within the above-mentioned range, the residue yield is high and thus is suitable for use. As an example, the sulfur-containing reducing agent may be used in an amount of 1 equivalent or more, 2 equivalents or more, 3 equivalents or more, or 4 equivalents or more, and 10 equivalents or less, 9 equivalents or less, 8 equivalents or less, or 7 equivalents or less, relative to 1 equivalent of the compound represented by Chemical Formula 1-3.

Further, the reduction reaction in the above step may be carried out in the presence of at least one base selected from the group consisting of potassium carbonate, sodium hydroxide, lithium hydroxide, potassium hydroxide, triethylamine, sodium hydrogen carbonate, potassium hydrogen carbonate, cesium carbonate, sodium carbonate, sodium methylate and potassium butyrate in terms of prevention of by-product generation, but the reduction reaction in the above step can be carried out even without a base.

As the reaction solvent in the above step, at least one selected from the group consisting of ethanol, methanol, isopropanol, butanol, tetrahydrofuran, acetone, dimethylformamide, methyl sulfoxide and acetonitrile may be used. This solvent may be used in an amount (mL/g) of 5 to 30 times (volume), more specifically in an amount (mL/g) of 5 to 15 times (volume), relative to the weight of the compound represented by Chemical Formula 1-3.

Preferably, the step 2 may be carried out by the steps of:
2-a) dissolving the compound represented by Chemical Formula 1-3 in the reaction solvent;
2-b) adding the above-mentioned base to the solution prepared in the step 2-a); and
2-c) adding the sulfur-containing reducing agent to perform a reduction reaction.

This reduction reaction can be carried out at 20 to 60°C for 1 hour to 2 hours. When the reaction is carried out in the above-mentioned temperature range, there are advantages that the color of the product hardly changes, impurities is not sharply increased in the above reaction time range, and all of the impurities generated can be removed through the work-up process.

Meanwhile, after the reaction is completed, without the step of purifying or isolating the prepared compound represented by Chemical Formula 1-4, the process up to step 2 and step 3 described hereinafter can be carried out in-situ in the same reaction vessel. As described above, since the step of purifying or isolating the compound is not required in step 2, the method for preparing the compound represented by Chemical Formula 1 including step 2 may be advantageous in terms of industrial mass production of the compound.

### Step 3)

Step 3 is a step of reacting a compound represented by Chemical Formula 1-4 with an orthoformate-based compound to prepare a compound represented by Chemical Formula 1-5. The reaction is preferably carried out in the presence of acid.

The orthoformate may be trimethyl orthoformate (TMOF), triethyl orthoformate, or diethyl phenyl orthoformate.

Further, the orthoformate-based compound may be used in an amount of 1 to 2 equivalents relative to 1 equivalent of the compound represented by Chemical Formula 1-4. When the orthoformate-based compound is used in an amount of less than 1 equivalent relative to 1 equivalent of the compound represented by Chemical Formula 1-4, there is a possibility that the reaction is not completed, which is not thus preferable. Even if the orthoformate-based compound is used in excess of 2 equivalents relative to 1 equivalent of the compound represented by Chemical Formula 1-4, the production yield does not substantially increase. As an example, the orthoformate-based compound may be used in an amount of 1 equivalent or more, 1.1 equivalents or more, or 1.2 equivalents or more, and less than 2 equivalents, less than 1.8 equivalents, less than 1.6 equivalents, or less than 1.5 equivalents, relative to 1 equivalent of the compound represented by Chemical Formula 1-4. Preferably, the orthoformate-based compound can be used in an amount of 1.3 equivalents relative to 1 equivalent of the compound represented by Chemical Formula 1-4.

Further, it is advantageous in terms of yield that the reaction in the above step is carried out in the presence of at least one acid catalyst selected from the group consisting of para-toluenesulfonic acid, methanesulfonic acid (MSA) and camposulfonic acid.

And, the reaction can be carried out at 40 to 60°C for 0.5 hour to 3 hours. When the reaction is carried out at a temperature lower than the above-mentioned temperature, there is a disadvantage in that the reaction rate is slow, and when the reaction is carried out at a temperature of 70 to 80°C, which is higher than the above reaction temperature, the reaction rate increases, but there is a possibility that impurities are generated.

Meanwhile, after the reaction is completed, the method may further comprise a step of crystallizing the reaction product with a non-polar organic solvent in order to purify the compound represented by Chemical Formula 1-5. For the crystallization, it is preferable to use a non-polar organic solvent in consideration of the solubility of the compound represented by Chemical Formula 1-5. When the reaction product is crystallized using a solvent in this way, it can be applied to industrialization, unlike the case where it is purified using a silica column, thereby being advantageous in terms of industrial mass production.

Specifically, as the non-polar organic solvent, normal hexane (n-hexane), which can increase the reaction yield and is less hazardous, may be used. At this time, the non-polar organic solvent may be used in an amount (mL/g) of 2 to 5 times (volume) relative to the weight of the compound represented by Chemical Formula 1-4. Further, the crystallization may be carried out by stirring at a temperature of about 0 to 10°C for 2 hours to 4 hours. If crystallization proceeds during the above-mentioned reaction time in the above-mentioned temperature range, the residual amount of product in the filtrate is lowest and the yield may be increased, which is thus preferred

### Step 4)

Step 4 is a step of reacting the compound represented by Chemical Formula 1-5 with hydrochloric acid in the presence of water and acetone to prepare the compound represented by Chemical Formula 1. Here, before reacting the compound represented by Chemical Formula 1-5 with hydrochloric acid in the presence of water and acetone, a deprotection reaction is carried out in the presence of an acid. Subsequently, as the deprotected compound is crystallized in the presence of water and acetone, 1HCl may be introduced into the deprotected compound to prepare the compound represented by Chemical Formula 1 which is the final compound.

The compound represented by Chemical Formula 1-5 contains two protecting groups P₁ and P₂, and a deprotection reaction of the two protecting groups is required to prepare the final compound. In Korean Patent No. 10-2084772, this deprotection reaction is carried out by adding an excessive amount of 4N hydrogen chloride dioxane solution to a tetrahydrofuran solvent and stirring the mixture at room temperature for 12 hours. In addition, it is produced in the form of dihydrochloride(2HCl) by the deprotection reaction. However, this method is not suitable for mass production as it requires excessive use of 4N hydrogen chloride dioxane solution which is expensive and dangerous and may vary in the concentration of hydrochloric acid depending on storage conditions. In addition, since the dihydrochloride(2HCl) form had problems such as hygroscopicity, preparation of the monohydrochloride(1HCl) form was required.

However, if 1) the deprotection reaction is carried out using low-cost hydrochloric acid along with an organic solvent, and then 2) 1HCl is introduced in the presence of hydrochloric acid, water, and acetone, not only the reaction time be reduced while greatly reducing the raw material cost, but also it is possible to directly prepare in monohydrochloride (1HCl) form, without the need to prepare the compound in dihydrochloride (2HCl) form into monohydrochloride (1HCl) form again, which thus makes it possible to apply it as an industrialization process..

Wherein, concentrated hydrochloric acid (c-HCl) may be used as the hydrochloric acid. For example, concentrated hydrochloric acid having a concentration of about 30 to 40% can be used in each of the deprotection reaction and the 1HCl introduction reaction.

Specifically, before reacting the compound represented by Chemical Formula 1-5 with hydrochloric acid in the presence of water and acetone, a deprotection reaction may be carried out in at least one organic solvent selected from the group consisting of ethyl acetate, dichloromethane, diethyl ether and tetrahydrofuran in the presence of hydrochloric acid. Among these, ethyl acetate is the preferred organic solvent in consideration of price and convenience of operation.

Further, the deprotection reaction may be carried out at 0 to 10°C for 30 minutes to 2 hours. If the reaction temperature is higher than 10°C, unknown impurities increase, which are not preferable. Since the reaction is completed within 2 hours, the above reaction time is preferred.

In addition, after the deprotection reaction, the method may further comprise a step of washing the reaction product with the same type of organic solvent used in the deprotection reaction. Thereby, impurities can be effectively removed, so that the purity of the final compound can be greatly improved.

Further, the 1HCl introduction reaction is a step of crystallizing (2R,3S)-2-(3-(4,5-dichloro-1H-benzo[d]imidazol-1-yl)propyl)piperidin-3-ol produced by the deprotection reaction into monohydrochloride form. Water and acetone may be used together as the crystallization solvent. This is because the cost of acetone is lower than other solvents such as methanol, ethanol, and tetrahydrofuran, and the use of acetone is preferred in that the final compound can be obtained with high quality and high yield, however, when the acetone is used alone, there is a possibility that in the process of forming 1HCl salt crystals, a phenomenon occurs in which they clump together.

In this case, the water and acetone may be used in a volume ratio of 1:10 to 1:40, or 1:15 to 1:25. While maintaining the quality of the product within the above-mentioned range, the yield of the product can be increased.

Further, hydrochloric acid used together with the water and acetone may be used in an amount of more than 0.5 equivalent and less than 2.0 equivalents relative to 1 equivalent of the compound represented by Chemical Formula 1-5. When the hydrochloric acid is used in an amount of 0.5 equivalent or less relative to 1 equivalent of the compound represented by Chemical Formula 1-5, there is a possibility that only about 50% of salt crystals is formed, and when the hydrochloric acid is used in excess of 2.0 equivalents relative to 1 equivalent of the compound represented by Chemical Formula 1-5, there is a problem in that it forms a compound in the 2HCl form and dissolves in water. Therefore, hydrochloric acid used with the water and acetone is preferably used in an amount of 0.9 to 1.1 equivalents, or 1 equivalent, relative to 1 equivalent of the compound represented by Chemical Formula 1-5.

The crystallization may be carried out at 0 to 10°C for 30 minutes to 4 hours. In the above-mentioned range, crystallization can be effectively carried out while minimizing the residual amount of the product in the above step.

### Step 5)

Meanwhile, after the step 4), the method may further comprise a step of purifying the compound represented by Chemical Formula 1 with purified water at a temperature of 70 to 80°C. The above step may effectively remove impurities and residual solvents and thus increase the purity of the final product.

In the above step, the quality and yield of the product can be improved by the high temperature slurry method when purified water is used instead of other organic solvents. At this time, the purified water may be used in an amount (mL/g) of 1 to 3 times (volume) relative to the weight of the compound represented by Chemical Formula 1.

Further, in order to effectively purify the compound represented by Chemical Formula 1, the compound represented by Chemical Formula 1 may be stirred in purified water at a temperature of 70 to 80°C for 30 minutes to 2 hours, and then further stirred at 0 to 5°C for 1 hour to 6 hours. When the reaction temperature is lower than 70°C, impurities and residual solvents is not removed yet, and when the reaction temperature is more than 80°C, unknown impurities increase, which is thus not preferable.

The method of preparing the compound represented by Chemical Formula 1 by the above-mentioned method not only can reduce the process steps but also can significantly improve the total yield as compared to the method described in Korean Patent No. 10-2084772, as can be seen in Examples and Comparative Examples described hereinafter. In addition, a crystallization purification method using a solvent instead of a column purification method is applied to each step, and high-risk or high-cost raw materials is replaced with low-cost and safe raw materials, whereby the above preparation method enables the mass production of the compound represented by Chemical Formula 1.

### (Method for preparing the compound represented by Chemical Formula 1-1)

Meanwhile, the compound represented by Chemical Formula 1-1, which is a starting material used in the method for preparing the compound represented by Chemical Formula 1, is prepared in accordance with the following Reaction Scheme 2: 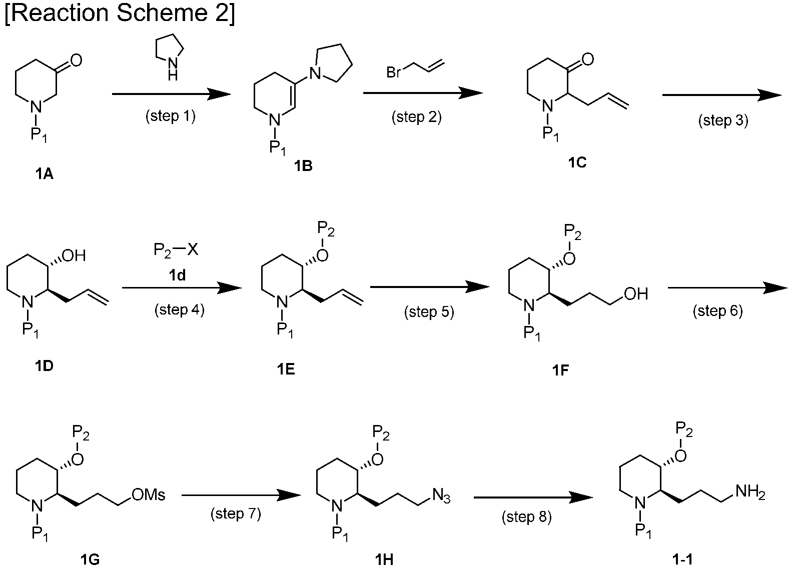 in Reaction Scheme 2,
X is halogen, Ms is methanesulfonyl, and P₁ and P₂ each independently represent a protecting group. Wherein the specific explanation of P₁ and P₂ is as described above, and X is preferably chloro or bromo.

### Step 1)

Step 1 is a step of reacting the compound represented by Chemical Formula 1A with pyrrolidine to prepare the compound represented by Chemical Formula 1B.

In the above step, the compound represented by Chemical Formula 1A and pyrrolidine may be used in an equivalent ratio of 1:0.8 to 1:2. Specifically, the compound represented by Chemical Formula 1A and pyrrolidine may be used in an equivalent ratio of 1:1.0 to 1:1.5, or 1:1.2.

The reaction can be carried out in an organic solvent such as toluene, dichloromethane, chloroform, dimethylformamide, dioxane, or tetrahydrofuran. Wherein the organic solvent may be used in an amount (L/kg) of 1 to 10 times (volume), more specifically in an amount(L/kg) of 2 to 4 times (volume), relative to the weight of the compound represented by Chemical Formula 1A.

Further, the reaction may be carried out at 80 to 150°C for 8 to 16 hours. The above-mentioned range is preferred in terms of reaction rate and preparation yield.

Meanwhile, after the reaction is completed, without the step of purifying or isolating the prepared compound represented by Chemical Formula 1B, the process up to step 1 and step 2 described hereinafter can be carried out in-situ in the same reaction vessel.

### Step 2)

Step 2 is a step of reacting the compound represented by Chemical Formula 1B with 3-bromo-1-propene to prepare the compound represented by Chemical Formula 1C. Through this step, substituents are introduced at the 2- and 3-positions of the piperidine ring.

In the above step, the compound represented by Chemical Formula 1B and 3-bromo-1-propene may be used in an equivalent ratio of 1:0.7 to 1:2. Specifically, the compound represented by Chemical Formula 1B and 3-bromo-1-propene may be used in an equivalent ratio of 1:0.7 to 1:1.3, 1:0.9 to 1:1.1, or 1:1.

The reaction can be carried out in a polar organic solvent. As an example, acetonitrile, methanol, tetrahydrofuran, acetone, dioxane, diethyl ether, dichloromethane, dimethylformamide, or the like may be used as the polar organic solvent. Wherein the polar organic solvent may be used in an amount (L/kg) of 1 to 10 times (volume), more specifically in an amount (L/kg) of 2 to 4 times (volume), relative to the weight of the compound represented by Chemical Formula 1B.

Further, the reaction may be carried out at 30 to 60°C, or 40 to 45°C for 8 hours to 16 hours. The above-mentioned range is preferred in terms of reaction rate and preparation yield.

### Step 3)

Step 3 is a step of reducing the compound represented by Chemical Formula 1C to prepare the compound represented by Chemical Formula 1D, wherein the reduction reaction is carried out in the presence of a ketoreductase enzyme. Therefore, through this step, the enzymatic asymmetric reduction of the ketone in the compound represented by Chemical Formula 1C proceeds, whereby the carbons at the 2- and 3-positions of the piperidine ring may become a chiral center. Specifically, the 2nd carbon of the piperidine ring in the compound represented by Chemical Formula 1D represents (R) chirality, and the 3rd carbon represents (S) chirality.

Meanwhile, Ketoreductase TJ-K066 available from Enzyme Works can be used as the ketoreductase enzyme.

The keto reductase enzyme may be used in an amount of 0.01 to 0.2 times (weight), more specifically in an amount of 0.05 to 0.15 times (weight), relative to the weight of the compound represented by Chemical Formula 1C.

Further, the keto reductase enzyme can be dissolved and used in a buffer solution. The buffer solution may be Na₂HPO₄ hydroxide, NaH₂PO₄ hydroxide, K₂HPO₄ hydroxide, or KH₂PO₄ hydroxide. For example, NaH₂PO₄·2H₂O, Na₂HPO₄·12H₂O, or a mixture thereof may be used as the buffer solution. At this time, it is preferable for the reaction that the buffer solution has a pH value of 6.5 to 7.5. This buffer solution may be used in an amount (L/kg) of 5 to 20 times (volume) relative to the weight of the compound represented by Chemical Formula 1C.

And, the reduction may be carried out in the presence of:
at least one enzyme selected from the group consisting of glutamate dehydrogenase (GDH) and glucose; and
at least one cofactor selected from the group consisting of nicotinamide adenine dinucleotide phosphate (NADP) and nicotinamide adenine dinucleotide (NAD).

At this time, the use of nicotinamide adenine dinucleotide phosphate (NADP) cofactor along with glutamic acid dehydrogenase (GDH)/glucose is preferred for the reduction reaction in terms of yield and quality.

Further, the reaction may be carried out under an inert gas at 20 to 40°C, or 25 to 30°C for 2 to 20 hours.

Meanwhile, after the reaction is completed, without the step of purifying or isolating the prepared compound represented by Chemical Formula 1D, the process up to step 3 and step 4 described hereinafter may be carried out in-situ in the same reaction vessel.

### Step 4)

Step 4 is a step of reacting the compound represented by Chemical Formula 1D with the compound represented by Chemical Formula 1d to prepare the compound represented by Chemical Formula 1E. Through this step, a protecting group is introduced into the hydroxy group of the piperidine ring.

In the above step, the compound represented by Chemical Formula 1D and the compound represented by Chemical Formula 1d may be used in an equivalent ratio of 1:1 to 1:2. Specifically, the compound represented by Chemical Formula 1D and the compound represented by Chemical Formula 1d may be used in an equivalent ratio of 1:1.2 to 1:2, 1:1.5 to 1:2, or 1:1.8.

Wherein, as the compound represented by Chemical Formula 1d, tert-butyldimethylsilyl chloride, 2-methoxyethoxymethyl ether chloride, methoxymethyl ether chloride, or the like may be used in the solution, but is not limited thereto.

Further, the reaction may be carried out in an organic solvent such as dichloromethane, toluene, chloroform, dimethylformamide, dioxane, or tetrahydrofuran. Wherein the organic solvent may be used in an amount (L/kg) of 1 to 10 times (volume), more specifically in an amount (L/kg) of 2 to 6 times(volume), relative to the weight of the compound represented by Chemical Formula 1D. Further, the reaction may be carried out at 0 to 25°C for 10 hours to 20 hours.

### Step 5)

Step 5 is a step of introducing a hydroxy group into the propenyl group at the 2-position of the compound represented by Chemical Formula 1E to prepare the compound represented by Chemical Formula F, which step is carried out by ① reacting the compound represented by Chemical Formula 1E with a borane-based compound, and ② reacting the prepared reactant with an oxidizing agent.

First, the compound represented by Chemical Formula 1E and the borane-based compound may be used in an equivalent ratio of 1:0.5 to 1:1.0. Specifically, the compound represented by Chemical Formula 1E and the borane-based compound may be used in an equivalent ratio of 1:0.5 to 1:1.0, or 1:0.7.

Wherein, the borane-based compound is used as a reducing agent, and a hydrogenation reaction of the compound represented by Chemical Formula 1E may proceed in the above step. As the borane-based compound, borane dimethylsulfide (BH₃-Me₂S) or borane tetrahydrofuran may be used, but is not limited thereto.

Further, the borane-based compound may be used in an amount of more than 0.5 equivalent and less than 1.0 equivalent relative to 1 equivalent of the compound represented by Chemical Formula 1E.

The reaction between the compound represented by Chemical Formula 1E and the borane-based compound may be carried out in an organic solvent such as tetrahydrofuran, dichloromethane, toluene, chloroform, dimethylformamide, or dioxane. Wherein the organic solvent may be used in an amount (L/kg) of 1 to 10 times (volume), more specifically in an amount (L/kg) of 2 to 6 times, relative to the weight of the compound represented by Chemical Formula 1E. Further, the reaction may be carried out at 5 to 10°C for 2 hours to 8 hours.

Next, the reactant between the compound represented by Chemical Formula 1E and the borane-based compound is subjected to an oxidation reaction in the presence of an oxidizing agent. As the oxidizing agent, at least one selected from the group consisting of hydrogen peroxide, Dess-Martin periodinane, and oxalyl chloride may be used. Further, the oxidation reaction may be carried out at 5 to 10°C for 10 hours to 20 hours.

### Step 6)

Step 6 is a step of reacting the compound represented by Chemical Formula 1F with methanesulfonyl chloride to prepare the compound represented by Chemical Formula 1G, wherein the reaction is carried out in the presence of a base, and a protecting group is introduced into the hydroxy group.

In the above step, the compound represented by Chemical Formula 1F and methanesulfonyl chloride may be used in an equivalent ratio of 1:0.7 to 1:1.5. Specifically, the compound represented by Chemical Formula 1F and methanesulfonyl chloride may be used in an equivalent ratio of 1:0.9 to 1:1.5, or 1:1.2.

Further, the base may be at least one selected from the group consisting of triethylamine, diisopropyl ethylamine, pyridine, dimethylaniline, dimethylamino pyridine, and sodium hydroxide. Wherein the base may be used in an amount of 1 to 2 equivalents, preferably 1 to 1.5 equivalents, relative to 1 equivalent of the compound represented by Chemical Formula 1F.

Further, the base may be at least one selected from the group consisting of triethylamine, diisopropyl ethylamine, pyridine, dimethylaniline, dimethylamino pyridine, and sodium hydroxide. At this time, the base may be used in an amount of 1 to 2 equivalents, preferably 1 to 1.5 equivalents, relative to 1 equivalent of the compound represented by Chemical Formula 1F.

Further, the reaction may be carried out in an organic solvent such as tetrahydrofuran, dichloromethane, toluene, chloroform, dimethylformamide, or dioxane. Wherein the organic solvent may be used in an amount (L/kg) of 1 to 10 times (volume), more specifically in an amount (L/kg) of 2 to 6 times (volume), relative to the weight of the compound represented by Chemical Formula 1F. Further, the reaction may be carried out at 0 to 5°C for 1 hour to 6 hours.

Meanwhile, after the reaction is completed, without the step of purifying or isolating the prepared compound represented by Chemical Formula 1G, the process up to step 6 and step 7 described hereinafter may be carried out in-situ in the same reaction vessel.

### Step 7)

Step 7 is a step of reacting the compound represented by Chemical Formula 1G with an azidation reagent to prepare the compound represented by Chemical Formula 1H. Through this reaction, the methanesulfonyl group of the compound represented by Chemical Formula 1G is substituted with an azide group.

As the azidation reagent, at least one selected from the group consisting of sodium azide, potassium azide, and trimethylsilyl azide may be used. Further, the azidation reagent may be used in an amount of 1 to 1.5 equivalents, preferably 1 to 1.3 equivalents, relative to 1 equivalent of the compound represented by Chemical Formula 1G.

Further, the reaction may be carried out in an organic solvent such as dimethylformamide, tetrahydrofuran, dichloromethane, toluene, chloroform, or dioxane. Wherein the organic solvent may be used in an amount (L/kg) of 1 to 10 times (volume), more specifically, in an amount (L/kg) of 2 to 6 times (volume), relative to the weight of the compound represented by Chemical Formula 1G. Further, the reaction may be carried out at 20 to 80°C for 8 hours to 16 hours in the presence of an inert gas such as nitrogen.

Meanwhile, after the reaction is completed, without the step of purifying or isolating the prepared compound represented by Chemical Formula 1H, the process up to step 7 and step 8 described hereinafter may be carried out in-situ in the same reaction vessel. The above preparation method may be advantageous in terms of industrial mass production of the compound because the four-step process of steps 1, 3, 6, and 7 is carried out in-situ and no additional separate isolation process is required at each step as described above.

### Step 8)

Step 8 is a step of reacting the compound represented by Chemical Formula 1H in the presence of a base to prepare the compound represented by Chemical Formula 1-1. Through this reaction, the azide group of the compound represented by Chemical Formula 1H is substituted with an amide group.

The base may be at least one selected from the group consisting of triphenyl phosphine, sodium borohydride, lithium aluminum hydride, and sodium hydride. Wherein the base may be used in an amount of 1 to 2 equivalents, preferably 1 to 1.2 equivalents, relative to 1 equivalent of the compound represented by Chemical Formula 1H.

Further, the reaction may be carried out in an ether-based organic solvent such as 2-methyltetrahydrofuran(2-MeTHF), diisopropyl ether, dioxane, or 1,2-dimethoxyethane. Wherein the organic solvent may be used in an amount (L/kg) of 1 to 10 times (volume), more specifically in an amount (L/kg) of 4 to 8 times (volume), relative to the weight of the compound represented by Chemical Formula 1H. Further, the reaction may be carried out at 5 to 25°C for 12 to 16 hours.

Meanwhile, after the reaction is completed, the method may further comprise a step of crystallizing the reaction product with acetonitrile in order to purify the compound represented by Chemical Formula 1-1. When the reaction product is crystallized with a solvent in this way, it can be applied to industrialization, unlike the case where the reaction product is purified using a silica column, thereby being advantageous in terms of industrial mass production.

The method of preparing the compound represented by Chemical Formula 1-1 by the above-mentioned method not only can reduce the process steps and dramatically shorten the process time but also can significantly improve the total yield, as compared to the method described in Korean Patent No. 10-2084772, as can be seen in Examples and Comparative Examples described hereinafter. In addition, a crystallization purification method using a solvent instead of a column purification method is applied to each step, whereby the above preparation method enables the mass production of the compound represented by Chemical Formula 1-1.

### (Method for preparing the compound represented by Chemical Formula 2)

Meanwhile, the compound represented by Chemical Formula 2, which can be used as an intermediate for preparing the compound represented by Chemical Formula 1-1, is prepared in accordance with the following Reaction Scheme 3: 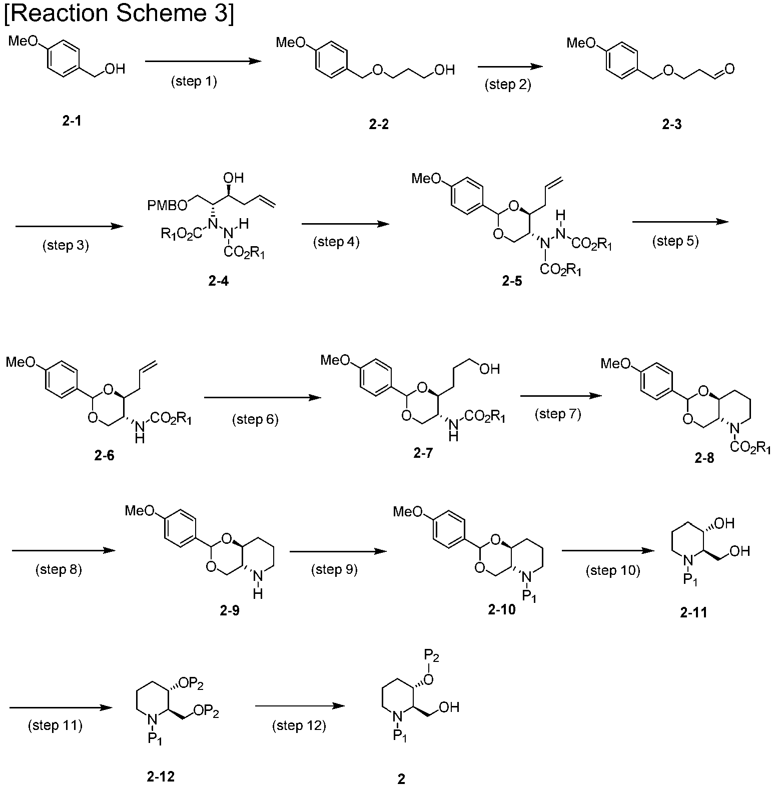 in Reaction Scheme 3,
R₁ is benzyl or a C₁₋₄ alkyl (linear or branched alkyl having 1 to 4 carbon atoms), and
P₁ and P₂ each independently represent a protecting group.

More specifically, P₁ and P₂ are as defined in Reaction Scheme 1. At this time, it is preferable in terms of ease of preparation and yield that P₁ is tert-butyloxycarbonyl (Boc), and P₂ is tert-butyldimethylsilyl (TBS).

### Step 1)

Step 1 is a step of reacting the compound represented by Chemical Formula 2-1 with propane-1,3-diol to prepare the compound represented by Chemical Formula 2-2, wherein the reaction is an etherification reaction, which is carried out in the presence of acid.

In the above step, the compound represented by Chemical Formula 2-1 and propane-1,3-diol may be used in an equivalent ratio of 1:0.5 to 1:2. Specifically, the compound represented by Chemical Formula 2-1 and propane-1,3-diol may be used in an equivalent ratio of 1:0.7 to 1:2, 1:1 to 1:1.8, or 1:1.5.

Further, as the acid used as a catalyst in the above step, a solid phase strong acid catalyst may be used. Specifically, a strong acid cation exchange resin can be used as the acid catalyst, and as such acid catalysts, Amberlyst^{®} 15, Amberlite^{®} IRC120 H, Amberlyst^{®} 16, Dowex^{®} 50W, and the like are commercially available.

### Step 2)

Step 2 is a step of oxidizing the compound represented by Chemical Formula 2-2 to prepare the compound represented by Chemical Formula 2-3, wherein the reaction is carried out in the presence of an alcohol oxidizing agent.

The oxidation proceeds in the presence of at least one oxidizing agent selected from the group consisting of pyridinium chlorochromate (PCC) and pyridinium dichlorochromate (PDC), or may proceed by SWERN oxidation reaction. Among these, the use of pyridinium chlorochromate is preferred in terms of reaction rate. In addition, the preparation of the compound represented by Chemical Formula 2-3 by SWERN oxidation is carried out by reacting the compound represented by Chemical Formula 2-2 with oxalyl chloride and dimethyl sulfoxide and then introducing an organic base such as triethylamine.

Further, the reaction may be carried out in an organic solvent such as dichloromethane (DCM), toluene, chloroform, dimethylformamide, dioxane, or tetrahydrofuran. Further, the reaction may be carried out at room temperature for 2 to 6 hours.

### Step 3)

Step 3 is a step of ① reacting the compound represented by Chemical Formula 2-3 with dibenzyl azodicarboxylate or di(C₁₋₄ alkyl)azodicarboxylate, and then ② reacting the reactant with allyl halide to prepare the compound represented by Chemical Formula 2-4.

First, a reaction proceeds in which the compound represented by Chemical Formula 2-3 is reacted with dibenzyl azodicarboxylate or di(C₁₋₄ alkyl)azodicarboxylate to introduce a dibenzyl hydrazine dicarboxylate group or a di(C₁₋₄ alkyl)hydrazine dicarboxylate group into the compound structure. In this step, the compound represented by Chemical Formula 2-3 and dibenzyl azodicarboxylate or di(C₁₋₄ alkyl)azodicarboxylate may be used in an equivalent ratio of 1:0.5 to 1:2.

Wherein, the dibenzyl azodicarboxylate or di(C₁₋₄ alkyl)azodicarboxylate may be selected from dibenzyl azodicarboxylate (DBAD), diethyl azodicarboxylate(DEAD), dibutyl azodicarboxylate and di-tert-butyl azodicarboxylate. Among these, it is preferable to use dibenzyl azodicarboxylate for proceeding a more stereoselective reaction.

Further, the reaction may proceed in the presence of a proline catalyst to increase the enantioselectivity of the reaction product while increasing the efficiency of the reaction between the compound represented by Chemical Formula 2-3 and the azodicarboxylate compound.

Further, the reaction may be carried out in an organic solvent such as acetonitrile, methanol, tetrahydrofuran, acetone, dioxane, diethyl ether, dichloromethane, or dimethylformamide at -20 to 20°C, preferably -5 to 5°C, more preferably 0°C, for 1 hour to 5 hours.

Next, a reaction proceeds in which the intermediate into which a dibenzyl hydrazinedicarboxylate group or a di(C₁₋₄ alkyl)hydrazinedicarboxylate group is introduced is reacted with allyl halide under a metal catalyst. Wherein, allyl bromide, alkyl chloride, and the like may be used as the allyl halide. This reaction can be carried out at -20 to 20°C, preferably -5 to 5°C, more preferably 0°C, for 30 minutes to 5 hours under a metal catalyst such as a zinc catalyst or an indium catalyst.

### Step 4)

Step 4 is a step of subjecting the compound represented by Chemical Formula 2-4 to a cyclization reaction to prepare the compound represented by Chemical Formula 2-5, which is carried out in the presence of an oxidizing agent.

The cyclization reaction may proceed in the presence of at least one oxidizing agent selected from the group consisting of 2,3-dichloro-5,6-dicyano-1,4-benzoquinone (DDQ), FeCl₃ and MoCl₅.

Further, the reaction may be carried out in an organic solvent such as dichloromethane (DCM), toluene, chloroform, dimethylformamide, dioxane, or tetrahydrofuran at -30 to 0°C for 4 hours to 10 hours. At this time, a molecular sieve may be used together to prevent moisture from flowing in during the reaction.

### Step 5)

Step 5 is a step of removing benzyl carbamate or C₁₋₄ alkyl carbamate from the compound represented by Chemical Formula 2-5 to prepare the compound represented by Chemical Formula 2-6, which is carried out in the presence of a base. Specifically, the benzyl carbamate or C₁₋₄ alkyl carbamate is degassed from the dibenzyl hydrazinedicarboxylate group or di(C₁₋₄ alkyl)hydrazinedicarboxylate group introduced in the step 3.

As the base, at least one selected from the group consisting of cesium carbonate, potassium carbonate, sodium hydroxide, lithium hydroxide, potassium hydroxide, triethylamine, sodium bicarbonate, potassium bicarbonate, sodium carbonate, sodium methylate and potassium butyrate may be used.

Further, the reaction may be carried out in an organic solvent such as acetonitrile (ACN), tetrahydrofuran (THF), 1,4-dioxane, N,N-dimethylformamide (DMF), and the like at 50 to 100°C for 20 hours to 30 hours.

### Step 6)

Step 6 is a step of subjecting the compound represented by Chemical Formula 2-6 to a boronation-oxidation reaction to prepare the compound represented by Chemical Formula 2-7. Wherein the hydroboration-oxidation reaction is a two-step hydration reaction that converts an alkene into an alcohol, and through the reaction, the alkene in the compound represented by Chemical Formula 2-6 may be converted into an alcohol.

First, the boronation reaction is carried out by a boronation reagent such as borane, borane dimethyl sulfide, and the like. At this time, the compound represented by Chemical Formula 2-6 and the boronation reagent may be used in an equivalent ratio of 1:1 to 1:1.5. Further, the reaction may be carried out in an organic solvent such as tetrahydrofuran, dioxane, diethyl ether, dichloromethane, or dimethylformamide at 0 to 25°C for 1 to 5 hours.

Next, the oxidation reaction may proceed in the presence of an oxidizing agent such as hydrogen peroxide, sodium perborate, 4-methylmorpholine N-oxide, or the like. The oxidation reaction may be carried out at 0 to 25°C for 1 hour to 5 hours.

### Step 7)

Step 7 is a step of subjecting the compound represented by Chemical Formula 2-7 to a cyclization reaction to prepare the compound represented by Chemical Formula 2-8, wherein the cyclization reaction may be carried out by reacting the compound represented by Chemical Formula 2-7 with methanesulfonyl chloride (MsCl) and then reacting the reactant with a strong base.

First, the reaction between the compound represented by Chemical Formula 2-7 and methanesulfonyl chloride (MsCl) may be carried out in the present of at least one base selected from the group consisting of triethylamine, pyridine, potassium carbonate and sodium carbonate at 0 to 25°C for 1 hour to 4 hours.

Next, the cyclization reaction may proceed in the presence of a strong base such as sodium hydride, potassium tertbutoxide, and sodium borohydride at -10 to 25°C for 1 hour to 4 hours to prepare the compound represented by Chemical Formula 2-8.

### Step 8)

Step 8 is a step of reacting the compound represented by Chemical Formula 2-8 in the presence of a base to prepare the compound represented by Chemical Formula 2-9. Through this step, a carboxylate group in the carbamate moiety can be removed.

The base may be at least one strong base selected from the group consisting of sodium hydroxide, lithium hydroxide, and potassium hydroxide. In addition, the reaction can be carried out in the presence of such a strong base for 3 hours to 10 hours.

### Step 9)

Step 9 is a step of introducing a protecting group into the compound represented by Chemical Formula 2-9 to prepare the compound represented by Chemical Formula 2-10, wherein the reaction is carried out in the presence of a base, through which a protecting group is introduced to the nitrogen atom of the amino group.

The compound represented by Chemical Formula 2-9 and the reagent capable of introducing a protecting group may be used in an equivalence ratio of 1:0.7 to 1:2. Specifically, the compound represented by Chemical Formula 2-9 and the reagent capable of introducing a protecting group may be used in an equivalent ratio of 1:0.9 to 1:1.8, or 1:1.5.

Further, the base may be at least one selected from the group consisting of triethylamine, diisopropyl ethylamine, pyridine, dimethylaniline, dimethylamino pyridine, and sodium hydroxide. At this time, the base may be used in an amount of 1 to 2.5 equivalents, preferably 1.5 to 2.0 equivalents, relative to 1 equivalent of the compound represented by Chemical Formula 2-9.

### Step 10)

Step 10 is a step of reacting the compound represented by Chemical Formula 2-10 in the presence of an acid to prepare the compound represented by Chemical Formula 2-11. Through this step, the para-methoxybenzyl group in the compound may be removed.

The acid may be at least one strong acid selected from the group consisting of camphorsulfonic acid (CSA) and p-toluene-4-sulfonic acid. Further, the reaction can be carried out in the presence of this strong acid for 20 hours to 40 hours.

### Step 11)

Step 11 is a step of introducing a protecting group into the compound represented by Chemical Formula 2-11 to prepare the compound represented by Chemical Formula 2-12, and the reaction is carried out in the presence of a base, through which a protecting group is introduced into the hydroxy group.

The compound represented by Chemical Formula 2-11 and the reagent capable of introducing a protecting group may be used in an equivalent ratio of 1:1.5 to 1:3.5. Specifically, the compound represented by Chemical Formula 2-11 and the reagent capable of introducing a protecting group may be used in an equivalent ratio of 1:2 to 1:3, or 1:2.5.

Further, the base may be at least one selected from the group consisting of imidazole, methylaminopyridine, and triethylamine. At this time, the base may be used in an amount of 2 to 4 equivalents, preferably 2.5 to 3.5 equivalents, relative to 1 equivalent of the compound represented by Chemical Formula 2-11.

### Step 12)

Step 12 is a step of reacting the compound represented by Chemical Formula 2-12 in the presence of an acid to prepare the compound represented by Chemical Formula 2. Through this step, some of the protecting groups in the compound may be removed.

The acid may be at least one strong acid selected from the group consisting of camphorsulfonic acid(CSA) and p-toluene-4-sulfonic acid. Further, the reaction may be carried out in the presence of this strong acid for 1 hour to 4 hours.

The compound represented by Chemical Formula 2 prepared through the above step may be used as an intermediate for preparing the compound represented by Chemical Formula 1-1. As an example, the compound represented by Chemical Formula 1-1 may be prepared using the compound represented by Chemical Formula 2 in accordance with the following Reaction Scheme 4: 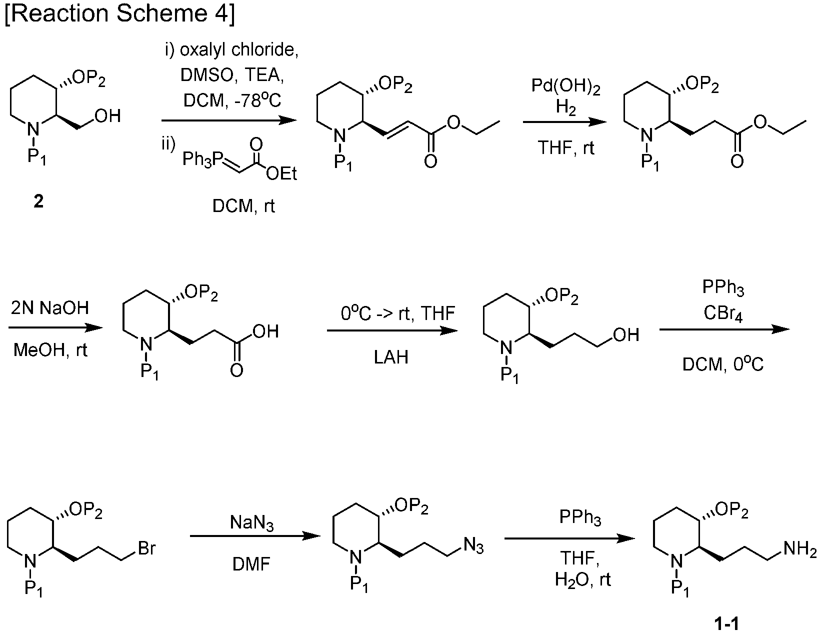 in Reaction Scheme 4, P₁ and P₂ each independently represent a protecting group, and more specifically, it is as defined in Reaction Scheme 1. Meanwhile, each step of Scheme 4 may be explained more specifically with reference to steps 13 to 19 of Comparative Example 2 described hereinafter.

### [Advantageous Effects]

As mentioned above, the preparation method according to the present invention has the advantage that (2R,3S)-2-(benzo[d]imidazolylpropyl)piperidin-3-ol derivative and its starting material can be prepared in a high yield even by the shortened process.

### [DETAILED DESCRIPTION OF THE EMBODIMENTS]

Below, the present invention will be described in more detail with reference to the following Examples. However, these examples are for illustrative purposes only, and the scope of the present invention is not limited thereby.

### Example 1-1: Preparation of the compound represented by Chemical Formula 1

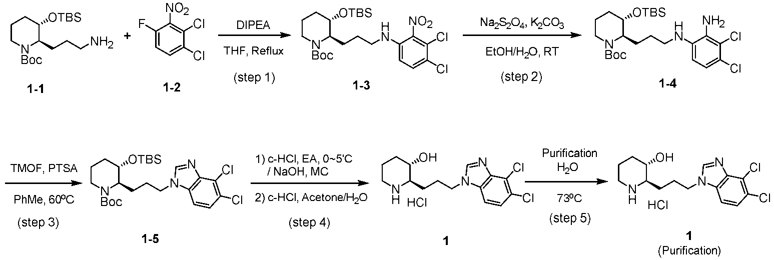

### Step 1) Preparation of Compounds 1-3

Compound 1-1, i.e., tert-butyl (2R,3S)-2-(3-aminopropyl)-3-((tert-butyldimethylsilyl)oxy)piperidine-1-carboxylate (100.0 g, 0.27 mol, 1.0 eq) was dissolved in 1000 mL of tetrahydrofuran, Compound 1-2, i.e., 1,2-dichloro-4-fluoro-3-nitrobenzene (56.4 g, 0.27 mol, 1.0 eq) and N,N-diisopropylethylamine (DIPEA, 93.5 mL, 0.54 mol, 2.0 eq) were added thereto, and the mixture was stirred under tetrahydrofuran reflux conditions (80-100°C) for 4 to 7 hours. When the reaction was completed, 1000 mL of purified water was added to extract the organic layer, to which 1000 mL of ethyl acetate was added and re-extracted. After completing the concentration under reduced pressure, 100 mL of ethanol was added to the concentrated residue, and re-concentrated. 400 mL of ethanol was added to the concentrated residue, and crystallization was performed. After crystals were formed, 400 mL of purified water was formed, and crystallization was carried out at 0-5°C for 2 hours. It was filtered under reduced pressure using a filter, and washed with a mixture of 100 mL of EtOH and 100 mL of purified water cooled to 0-5°C. This was vacuum-dried at 45-55°C for 12 hours to afford Compound 1-3, i.e., tert-butyl (2R,3S)-3-((tert-butyldimethylsilyl)oxy)-2-(3-((3,4-dichloro-2-nitrophenyl)amino)propyl)piperidine-1-carboxylate as an orange or red solid (131.5 g, yield: 87%).
¹H NMR (500 MHz, MeOD): δ 7.43 (d, 1H), 6.89 (d, 1H), 4.08 (s, 1H), 3.94 (s, 1H), 3.76 (s, 1H), 3.24 (m, 2H), 2.76 (s, 1H), 1.86 (m, 1H), 1.74 (m, 2H), 1.58 (m, 3H), 1.48 (s, 10H), 1.45 (s, 1H), 0.90 (s, 9H), 0.07 (d, 6H)

### Step 2) Preparation of Compound 1-4

After Compound 1-3 (120.0 g, 0.21 mol, 1.0 eq) obtained in step 1 was dissolved in 1200 mL of ethanol, potassium carbonate (176.9 g, 1.28 mol, 6.0 eq) was added thereto and 1400 mL of sodium hydrosulfite aqueous solution (content of Na₂S₂O₄ in aqueous solution: 222.8 g, 1.28 mol, 6.0 eq) was added, and then stirred at room temperature for 1 hour. When the reaction was completed, ethanol was concentrated under reduced pressure, and then 600 mL of purified water and 1,200 mL of ethyl acetate were added and extracted. 600 mL of ethyl acetate was added and re-extracted. The organic layer was washed with 1200 mL of brine. Sodium sulfate was added for moisture drying, and the excess moisture was removed. This was concentrated under reduced pressure to afford Compound 1-4, i.e., tert-butyl (2R,3S)-2-(3-((2-amino-3,4-dichlorophenyl)amino)propyl)-3-((tert-butyldimethylsilyl)oxy)piperidine-1-carboxylate as a brown liquid (113.6 g, yield: 100%). The resulting compound was used in the next step without purification.

### Step 3) Preparation of Compounds 1-5

After Compound 1-4 (113.6 g, 0.21 mol, 1.0 eq) obtained in step 2 was dissolved in 1136 mL of toluene, trimethyl orthoformate (TMOF, 30.3 mL, 0.28 mol, 1.3 eq) and para-toluenesulfonilic acid (0.4 g, 0.02 mol, 0.1 eq) were added thereto, and stirred at 50-60°C for 1~2 hours. When the reaction was completed, 1136 mL of toluene used in the reaction was removed by concentration under reduced pressure. 122 mL of sodium bicarbonate aqueous solution, 1136 mL of ethyl acetate, and 1136 mL of purified water were added and extracted. At this time, layer isolation was easily performed. Then, 568 mL of EA was added to the aqueous layer and re-extracted. The organic layer was treated with activated carbon (11.4 g, 0.1 eq) to remove color, and stirred for 15 minutes. Sodium sulfate was added to remove moisture, and stirred for 15 minutes, and filtered through celite. After completing the concentration of the filtrate under reduced pressure, 227 mL of normal hexane was added to the concentrated residue, concentrated under reduced pressure. 340 mL of normal hexane was added thereto, and the mixture was stirred at reflux for 30 minutes to dissolve the crystals, cooled to 0-5°C, and then stirred at the same temperature for 4 hours. The reaction mixture was filtered under reduced pressure using a filter, and washed with 113 mL of normal hexane cooled to 0-5°C. This was vacuum-died at 45-55°C for 12 hours to afford Compounds 1-5, i.e., tert-butyl (2R,3S)-3-((tert-butyldimethylsilyl)oxy)-2-(3-(4,5-dichloro-1H-benzo[d]imidazol-1-yl)propyl)piperidine-1-carboxylate as a white solid (100 g, yield: 86%). In addition, the filtrate was concentrated and the crystallization process is carried out in the same manner to further obtain the compound 1-5 as a white solid (8.0 g, yield: 8%), thereby obtaining the final compound 1-5 (108 g, yield: 94%).
¹H NMR (500 MHz, MeOD): δ 8.30 (s, 1H), 7.56 (d, 1H), 7.43 (d, 1H), 4.30 (m, 2H), 4.17 (s, 1H), 4.05 (s, 1H), 3.91 (d, 1H), 3.73 (s, 1H), 2.68 (s, 1H), 1.87 (s, 3H), 1.70 (t, 2H), 1.55 (d, 1H), 1.45 (m, 10H), 1.42 (s, 1H), 0.90 (s, 9H), 0.07 (d, 6H)

### Step 4) Preparation of the compound represented by Chemical Formula 1

Compound 1-5 (90.0 g, 0.17 mol, 1.0 eq) obtained in step 3 was dissolved in 540 mL of ethyl acetate and cooled to 0-10°C. Concentrated hydrochloric acid (146.4 mL, 10.0 eq) was added thereto, and stirred for 1~2 hours. After the reaction was completed, 540 mL of purified water was added thereto, and extracted at room temperature (present in the aqueous layer of the product under acid conditions). 540 mL of ethyl acetate was added again to the aqueous layer, and re-extracted (impurities were removed with EA). The organic layer was discarded, and 8N aqueous sodium hydroxide solution was slowly added to adjust the pH to 12.5 or higher. 900 mL of dichloromethane was added, and extracted (present in the product MC layer under basic conditions). 450 mL of dichloromethane was added, and re-extracted. After completing the concentration under reduced pressure, 1100 mL of acetone and 54 mL of purified water were added to and dissolved in the concentrated residue, i.e., freebase (2R,3S)-2-(3-(4,5-dichloro-1H-benzo[d]imidazol-1-yl)propyl)piperidin-3-ol. 1 Equivalent of concentrated hydrochloric acid was added dropwise in 3 parts. The resulting crystals were stirred at 0-5°C for 4 hours to proceed with crystallization. The reaction mixture was filtered under reduced pressure using a filter, and washed with 109 mL of acetone cooled to 0-5°C. This was vacuum-dried at 45-55°C for 12 hours to afford the compound represented by Chemical Formula 1, i.e., (2R,3S)-2-(3-(4,5-dichloro-1H-benzo[d]imidazol-1-yl)propyl)piperidin-3-ol 1HCl as a white solid (58.5 g, yield: 97%).
¹H NMR (500 MHz, DMSO): δ 8.45 (s, 1H), 7.71 (d, 1H), 7.47 (d, 1H), 5.40 (d, 1H), 4.32 (m, 2H), 3.41 (m, 1H), 3.08 (d, 1H), 2.75 (m, 2H), 2.07 (m, 1H), 1.97 (m, 1H), 1.85 (m, 2H), 1.75 (m, 1H), 1.65 (m, 1H), 1.52 (m, 1H), 1.35 (m, 1H)

### Step 5) Further purification process

110 mL of purified water was added to the compound represented by Chemical Formula 1 (55 g, 0.15 mol, 1.0 eq) obtained in step 4, stirred at 70-75°C for 1 hour, cooled to 0-5°C, and then stirred at the same temperature for 4 hours. The mixture was filtered under reduced pressure using a filter, and washed with 55 mL of acetone cooled to 0-5°C. This was vacuum-dried at 45-55°C for 12 hours to afford the compound represented by Chemical Formula 1 as a purified white solid (52.0 g, yield: 94%).
¹H NMR (500 MHz, DMSO): δ 8.45 (s, 1H), 7.71 (d, 1H), 7.47 (d, 1H), 5.40 (d, 1H), 4.32 (m, 2H), 3.41 (m, 1H), 3.08 (d, 1H), 2.75 (m, 2H), 2.07 (m, 1H), 1.97 (m, 1H), 1.85 (m, 2H), 1.75 (m, 1H), 1.65 (m, 1H), 1.52 (m, 1H), 1.35 (m, 1H)

### Example 1-2: Preparation of the compound represented by Chemical Formula 1

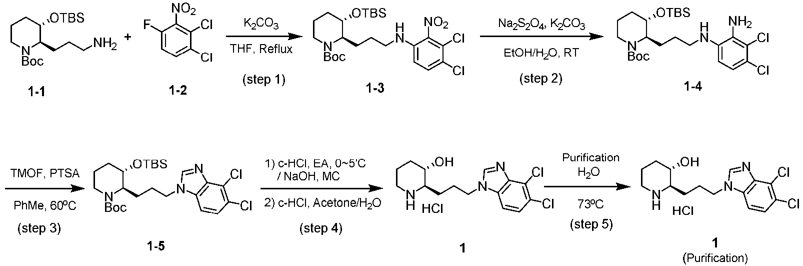

### Step 1) Preparation of Compounds 1-3

Compound 1-1, i.e., tert-butyl (2R,3S)-2-(3-aminopropyl)-3-((tert-butyldimethylsilyl)oxy)piperidine-1-carboxylate (100.0 g, 0.27 mol, 1.0 eq) was dissolved in 1000 mL of tetrahydrofuran, and Compound 1-2, i.e., 1,2-dichloro-4-fluoro-3-nitrobenzene (56.4 g, 0.27 mol, 1.0 eq) and potassium carbonate (K₂CO₃, 74.2g, 0.54 mol, 2.0 eq) were added thereto, and stirred under tetrahydrofuran reflux conditions (80 to 100°C) for 1~3 hours. When the reaction was completed, 1000 mL of purified water was added to extract the organic layer. 1000 mL of ethyl acetate was added and re-extracted. After completing the concentration under reduced pressure, 100 mL of ethanol was added to the concentrated residue, and re-concentrated. 400 mL of ethanol was added to and dissolved in the concentrated residue, and then crystallization was carried out. After crystals were formed, 400 mL of purified water was added, and crystallized at 0-5°C for 2 hours. The reaction mixture was filtered under reduced pressure using a filter, and washed with a mixture of 100 mL of EtOH and 100 mL of purified water cooled to 0-5°C. This was vacuum-dried at 45-55°C for 12 hours to afford Compound 1-3, i.e., tert-butyl (2R,3S)-3-((tert-butyldimethylsilyl)oxy)-2-(3-((3,4-dichloro-2-nitro-phenyl)amino)propyl)piperidine-1-carboxylate as an orange or red solid (143.0 g, yield: 95%).
¹H NMR (500 MHz, MeOD): δ 7.43 (d, 1H), 6.89 (d, 1H), 4.08 (s, 1H), 3.94 (s, 1H), 3.76 (s, 1H), 3.24 (m, 2H), 2.76 (s, 1H), 1.86 (m, 1H), 1.74 (m, 2H), 1.58 (m, 3H), 1.48 (s, 10H), 1.45 (s, 1H), 0.90 (s, 9H), 0.07 (d, 6H)

### Steps 2) to 4)

Subsequently, steps 2) to 4) were performed in the same manner as in Example 1-1 to afford (2R, 3S)-2-(3-(4,5-dichloro-1H-benzo[d]imidazol-1-yl)propyl)piperidin-3-ol 1HCl (58.5 g, yield: 97%).
¹H NMR (500 MHz, DMSO): δ 8.45 (s, 1H), 7.71 (d, 1H), 7.47 (d, 1H), 5.40 (d, 1H), 4.32 (m, 2H), 3.41 (m, 1H), 3.08 (d, 1H), 2.75 (m, 2H), 2.07 (m, 1H), 1.97 (m, 1H), 1.85 (m, 2H), 1.75 (m, 1H), 1.65 (m, 1H), 1.52 (m, 1H), 1.35 (m, 1H)

### Step 5) Further purification process

110 mL of purified water was added to the compound represented by Chemical Formula 1 (55 g, 0.15 mol, 1.0 eq) obtained in step 4, stirred at 70-75°C for 1 hour, cooled to 0-5°C, and stirred at the same temperature for 4 hours. The reaction mixture was filtered under reduced pressure using a filter, washed with 55 mL of acetone cooled to 0-5°C. This was vacuum-dried at 45-55°C for 12 hours to afford the compound represented by Chemical Formula 1 as a purified white solid (52.0 g, yield: 94%).
¹H NMR (500 MHz, DMSO): δ 8.45 (s, 1H), 7.71 (d, 1H), 7.47 (d, 1H), 5.40 (d, 1H), 4.32 (m, 2H), 3.41 (m, 1H), 3.08 (d, 1H), 2.75 (m, 2H), 2.07 (m, 1H), 1.97 (m, 1H), 1.85 (m, 2H), 1.75 (m, 1H), 1.65 (m, 1H), 1.52 (m, 1H), 1.35 (m, 1H)

### Comparative Example 1: Preparation of the compound represented by Chemical Formula 1

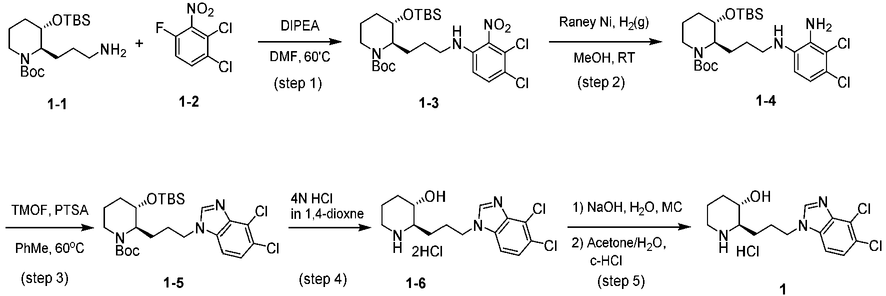

### Step 1) Preparation of Compounds 1-3

Compound 1-1, i.e., tert-butyl (2R,3S)-2-(3-aminopropyl)-3-((tert-butyldimethylsilyl)oxy)piperidine-1-carboxylate (1.0 g, 2.7 mmol) was added to and dissolved in N,N-dimethylformamide (25 mL, 0.67 M), and then Compound 1-2, i.e., 1,2-dichloro-4-fluoro-3-nitrobenzene (629.97 mg, 3.0 mmol) and N,N-diisopropylethylamine (0.93 mL, 5.4 mmol) were added thereto, and heated and stirred at 60°C for 2 hours. When the reaction was completed, the reaction mixture was diluted with ethyl acetate, and washed with saturated sodium chloride solution. The organic layer was collected, dried over sodium sulfate, filtered, concentrated under reduced pressure, and then purified by a column chromatography (hexane:ethyl acetate=5:1) to afford Compound 1-3 (1.2 g, yield: 81%).
¹H NMR (500 MHz, MeOD): δ 7.43 (d, 1H), 6.89 (d, 1H), 4.08 (s, 1H), 3.94 (s, 1H), 3.76 (s, 1H), 3.24 (m, 2H), 2.76 (s, 1H), 1.86 (m, 1H), 1.74 (m, 2H), 1.58 (m, 3H), 1.48 (s, 10H), 1.45 (s, 1H), 0.90 (s, 9H), 0.07 (d, 6H)

### Step 2) Preparation of Compounds 1-4

Compound 1-3 (2.6 mg, 4.9 mmol) obtained in step 1 was dissolved in methanol (25 mL, 0.2 M), and then an appropriate amount of Raney nickel was added thereto. After connecting a hydrogen balloon, the mixture was stirred at room temperature for 1 hour. When the reaction was completed, the reaction solution was filtered through celite, and concentrated under reduced pressure to afford Compound 1-4. The resulting compound was used in the next step without purification.

### Step 3) Preparation of Compounds 1-5

After Compound 1-4 obtained in step 2 was added to and dissolved in toluene (30 mL, 0.16 M), trimethyl orthoformate (1.6 mL, 14.6 mmol) and p-toluenesulfonic acid (168 mg, 0.98 mmol) were added thereto, heated and stirred at 50°C for 6 hours. When the reaction was completed, the solvent was removed, diluted with ethyl acetate, and washed with saturated sodium chloride solution. The organic layer was collected, dried with magnesium sulfate, filtered, concentrated under reduced pressure, and then purified by a column chromatography (hexane:ethyl acetate=1:1) to afford Compound 1-5 (1.8 g, yield: 74%).
¹H NMR (500 MHz, MeOD): δ 8.30 (s, 1H), 7.56 (d, 1H), 7.43 (d, 1H), 4.30 (m, 2H), 4.17 (s, 1H), 4.05 (s, 1H), 3.91 (d, 1H), 3.73 (s, 1H), 2.68 (s, 1H), 1.87 (s, 3H), 1.70 (t, 2H), 1.55 (d, 1H), 1.45 (m, 10H), 1.42 (s, 1H), 0.90 (s, 9H), 0.07 (d, 6H)

### Step 4) Preparation of Compounds 1-6

After Compound 1-5 (1.8 g, 3.6 mmol) obtained in step 3 was dissolved in a small amount of tetrahydrofuran, 4N hydrogen chloride dioxane solution (30 mL, 0.12 M) was added thereto, and stirred at room temperature for 12 hours. When the reaction was completed, the reaction solution was concentrated under reduced pressure to remove the solvent, a small amount of methanol was added and dissolved, and then crystallized with diethyl ether to afford Compound 1-6, i.e., (2R,3S)-2-(3-(4,5-dichloro-1H-benzo[d]imidazol-1-yl)propyl)piperidin-3-ol 2HCl (26 mg, yield: 81%).
¹H NMR (500 MHz, MeOD): δ 9.67 (s, 1H). 8.02 (d, 1H), 7.82 (d, 1H), 4.62 (m, 2H), 3.60 (m, 1H), 3.28 (m, 1H), 2.99 (m, 2H), 2.25 (m, 2H), 2.08 (m, 2H), 1.99 (m, 1H), 1.78 (m, 2H), 1.54 (m, 1H)

### Step 5) Preparation of the compound represented by Chemical Formula 1

Compound 1-6 (17.3 g, 0.04 mol, 1.0eq) obtained in step 4 was dissolved in 86.5 mL of an aqueous solution in which sodium hydroxide (NaOH, 6.9 g, 0.16 mol, 4.0 eq) was dissolved. This was added to 346 mL of dichloromethane, extracted, and then the aqueous layer was re-extracted with 173 mL of dichloromethane. Sodium sulfate was added to dry the moisture to remove the excess moisture. After completing the concentration under reduced pressure, the mixture was dissolved in 346 mL of acetone and 17.3 mL of purified water. 1 Equivalent of concentrated hydrochloric acid (pH 6.5-7) was added, and stirred at room temperature for 2 hours. The resulting mixture was filtered under reduced pressure using a filter to afford a white solid. 16 mL of purified water was added thereto, stirred at 75°C for 1 hour, and then stirred at 5°C for 1 hour. The rection mixture was filtered under reduced pressure using a filter to afford the title compound represented by Chemical Formula 1 as a purified white solid (11.1 g, yield: 71%).
¹H NMR (500 MHz, DMSO): δ 8.45 (s, 1H), 7.71 (d, 1H), 7.47 (d, 1H), 5.40 (d, 1H), 4.32 (m, 2H), 3.41 (m, 1H), 3.08 (d, 1H), 2.75 (m, 2H), 2.07 (m, 1H), 1.97 (m, 1H), 1.85 (m, 2H), 1.75 (m, 1H), 1.65 (m, 1H), 1.52 (m, 1H), 1.35 (m, 1H)

### Comparison of Example 1-1, Example 1-2 and Comparative Example 1

In order to compare the case where the compound represented by Chemical Formula 1 was prepared by the preparation method of Examples 1-1 and 1-2 with that case where it was prepared by that of Comparative Example 1, the yield of each preparation method was summarized in Table 1 below.

**[Table 1]**

| | Example 1-1 | Example 1-2 | Comparative Example 1 |
|---|---|---|---|
| Number of process step | 4 steps | 4 steps | 5 steps |
| Yield of each step | Step 1) 87% | Step 1) 95% | Step 1) 81% |
| | Step 2) 100% (in-situ) | Step 2) 100% (in-situ) | Step 2) 100% (in-situ) |
| | Step 3) 94% | Step 3) 94% | Step 3) 74% |
| | Step 4) 97% | Step 4) 97% | Step 4) 81% |
| | | | Step 5) 71% |
| Final yield | 79.3% | 86.6% | 34.5% |

As shown in Table 1, it is confirmed that the preparation method of Examples can prepare the compound represented by Chemical Formula 1 with improved yield while shortening the process steps as compared to the preparation method of Comparative Example 1.

Furthermore, it is confirmed that the preparation method of Examples not only does not use highly dangerous or expensive raw materials in each process step, but also does not use a column purification method that is difficult to apply to industrialization, which is thus suitable for application to mass production of the compound represented by Chemical Formula 1.

### Example 2: Preparation of Compound 1-1 (tert-butyl (2R, 3S)-2-(3-aminopropyl)-3-((tert-butyldimethylsilyl)oxy)piperidine-1-carboxylate)

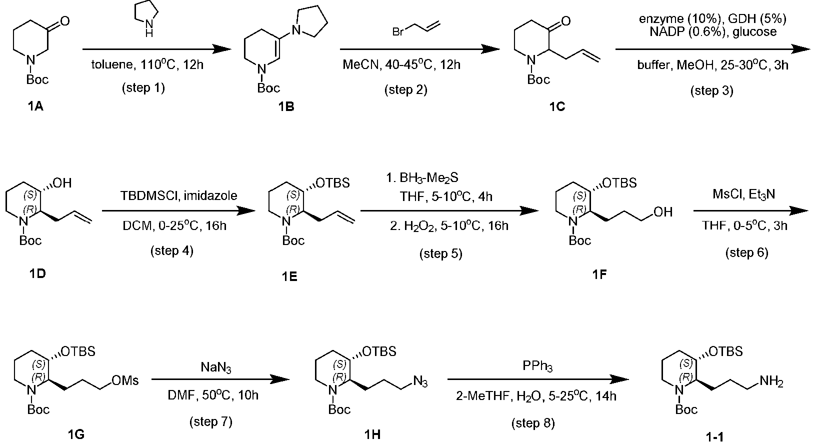

### Step 1) Preparation of Compound 1B

To a solution in which Compound 1A (180 kg, 903.4 mol, 1.00 eq) was dissolved in toluene (540 L), pyrrolidine (77.1 kg, 1.08 kmol, 90.5 L, 1.20 eq) was added at 15-25°C. The prepared mixture was stirred at 110°C for 12 hours using a Dean-Stark trap. It was confirmed by TLC (petroleum ether/ethyl acetate = 2/1, Rf = 0.56) that Chemical Formula 1A was consumed completely and one new spot was formed. The prepared reactant was concentrated in vacuum to afford Compound 1B (228.0 kg, crude) as a black oil. The resulting compound was used in the next step without purification.

### Step 2) Preparation of Compound 1C

To a solution in which Compound 1B (228 kg, 903 mol, 1.00 eq) obtained in step 1 was dissolved in MeCN (680 L), 3-bromo-1-propene (109.2 kg, 903 mol, 1.00 eq) was added dropwise over 5 hours at 40-45°C. The prepared mixture was stirred at 40-45°C for 12 hours. It was confirmed by TLC (petroleum ether/ethyl acetate = 2/1, Rf = 0.13) that the compound 1B was consumed completely. The prepared reactant was cooled to 15-20°C. Then, the reactant was poured into HCl (750 L, 0.5 N), and extracted with methyl tertiary-butyl ether (MTBE, 500 L, 200 L). The combined organic phase was washed with water (200 L) and brine (200 L), dried with anhydrous Na₂SO₄, and then filtered and concentrated under vacuum conditions. The resulting residue was purified by a silica gel chromatography (1 m x 2 m column, eluent: petroleum ether/ethyl acetate = 4/1). The wet product was confirmed by gas chromatography (GC) and high performance liquid chromatography (HPLC). Thereby, Compound 1C as a yellow oil (150 kg, 600 mol, yield: 66.41%, purity: 95.4%) was obtained, and confirmed by ¹H NMR.
¹H NMR (400 MHz CDCl₃): 5.65-5.75 (m, 1H), 5.01-5.06 (m, 2H), 4.50-4.51 (m, 1H), 3.10-3.11 (m, 1H), 2.39-2.47 (m, 4H), 1.90-1.94 (m, 2H), 1.41(s, 9H)

### Step 3) Preparation of Compound 1D

Compound 1C (150 kg, 627 mol, 1.00 eq) obtained in step 2, nicotinamide adenine dinucleotide phosphate (NADP, 900 g), glutamate dehydrogenase (GDH, 7.5 kg), glucose (225 kg, 1.14 kmol, 1.81 eq), an enzyme (Ketoreductase TJ-K066 from Enzyme Works, 15 kg) in a buffer (Na₂HPO₄·12H₂O 33.3 kg, NaH₂PO₄·2H₂O in water (1500 L) 8.88 kg, pH = 7.0, 1500 L), and MeOH (150 L) were mixed, and the resulting mixture was stirred at 25-30°C for 3 hours under N₂ atmosphere. The pH value of the mixture was adjusted to 6.0-6.5 using 1N NaOH. As a result of confirming the reaction by chiral GC (product: RT=34.7 mins, reactant: RT=35.7 mins), it was found that 42.1% of the product was formed, and 49.6% of the starting material remained. The reaction mixture was heated to 60°C for 1 hour, and then filtered. The filter cake was washed with H₂O (100 L). The reactant was filtered, and the aqueous phase was extracted with MTBE (600 L, 300 L, 200 L). The organic phase was washed with brine (200 L), dried over anhydrous Na₂SO₄, then filtered and concentrated under vacuum conditions. The resulting product, i.e., Compound 1D (124 kg, crude) as a yellow oil was used directly in the next step. At this time, the purity was confirmed through chiral GC, HPLC, and supercritical fluid chromatography (SFC).

### Step 4) Preparation of Compound 1E

To a solution in which Compound 1D (124 kg, 235 mol, 1.00 eq) obtained in step 3 and imidazole (35.2 kg, 516 mol, 2.2 eq) were dissolved in DCM (500 L), tert-butyldimethylsilyl chloride (TBDMSCl, 63.7 kg, 423 mol, 1.80 eq) was added at 0°C. The prepared mixture was stirred at 0-25°C for 16 hours. It was confirmed by TLC (petroleum ether/ethyl acetate = 2/1, Rf = 0.8) that Compound 1D was consumed completely and one new major spot was detected. The prepared reactant was washed with water (200 L). Then, the organic layer was dried, and concentrated under reduced pressure to afford a black oil. This black oil was purified by column chromatography (SiO₂, petroleum ether/ethyl acetate = 150/1 to 1/1) to afford Compound 1E as a yellow oil (96.5 kg, 52.8% yield, crude).
¹H NMR (400 MHz CDCl₃): δ 5.66-5.76 (m, 1H), 4.94-5.04 (m, 2H), 3.71-4.10 (m, 2H), 2.70-2.74 (m, 1H), 2.25-2.29 (m, 2H), 1.90 (m, 1H), 1.59-1.63 (m, 2H), 1.41 (s, 9H), 1.27-1.30 (m, 2H), 0.86 (s, 9H), 0.01 (d, J = 4.0 Hz, 6H)

### Step 5) Preparation of Compound 1F

In 24 batches, the following reactions were carried out in parallel (4 kg x 23, 3 kg x 1).

To a solution in which Compound 1E (4.00 kg, 11.3 mol, 1.00 eq) obtained in step 5 was dissolved in THF (16 L), BH₃-Me₂S (10 M, 787 mL, 0.7 eq) was added. The prepared mixture was stirred at 5-10°C for 4 hours. Then, EtOH (3.00 L) and NaOH (3 M, 11.3 L, 3.00 eq) were added to the mixture at 5-10°C. H₂O₂ (5.10 kg, 45.0 mol, 4.32 L, purity: 30%, 4.00 eq) was added thereto at 5~10°C for 16 hours. The reaction product was confirmed by HPLC. It was confirmed by TLC (petroleum ether/ethyl acetate = 2/1, Rf = 0.25) that Compound 1E was consumed completely and many new spots were detected. The reaction mixture was isolated, and the aqueous phase was extracted with MTBE (3.00 L, 2.00 L). The combined organic phase was washed with saturated NaHSO₃ (5.0 L, 500 g). The mixture was then isolated, and the organic phase was dried and concentrated under reduced pressure to afford a yellow oil.

The 24 batch products having reaction completed were combined, and then purified by column chromatography (petroleum ether/ethyl acetate=300/1 to 1/2) to afford a yellow oil. Finally, Compound 1F (41 kg, yield: 42.4%) was obtained.
¹H NMR (400 MHz CDCl₃): δ 4.05-4.12 (m, 2H), 3.61-3.67 (m, 3H), 2.66-2.72 (m, 1H), 1.87-1.90 (m, 1H), 1.51-1.63 (m, 6H), 1.41 (s, 9H), 1.23-1.25 (m, 2H), 0.85 (s, 9H), 0.01-0.03(d, J = 4.0 Hz, 6H)

### Step 6) Preparation of Compound 1G

In 9 batches, the following reactions were carried out in parallel.

To a solution in which Compound 1F (4.50 kg, 12.0 mol, 1.00 eq) obtained in step 5 was dissolved in THF (18.0 L), triethylamine (TEA, 1.83 kg, 18.1 mol, 2.60 L, 1.50 eq) and methanesulfonyl chloride (MsCl, 1.66 kg, 14.5 mol, 1.15 L, 1.20 eq) were added at 0°C for 1 hour. The prepared mixture was stirred at 0-5°C for 2 hours. It was confirmed by TLC (petroleum ether/ethyl acetate = 2/1, Rf = 0.5) that the compound 1F was consumed completely. The reaction product was cleared up by TLC. The mixture was filtered, and the filter cake was washed with MTBE (20 L).

The reaction filtrates obtained in 9 batches were combined, and then concentrated to afford Compound 1G in the form of a yellow solution, which was used in the next step without further purification.
¹H NMR (400 MHz CDCl₃): δ 4.16 - 4.30 (m, 2H), 3.97 - 4.14 (m, 2H), 3.65 (br d, J = 1.2 Hz, 1H), 2.98 (s, 3H), 2.66 (br t, J = 12.4 Hz, 1H), 1.85 - 1.94 (m, 1H), 1.50 - 1.77 (m, 6H), 1.41 (s, 10H), 1.28-1.32 (m, 1H), 0.85 (s, 9H), 0.03 (s, 3H), 0.00 (s, 3H)

### Step 7) Preparation of Compound 1H

In 12 batches, the following reactions were carried out in parallel.

To a solution in which Compound 1G (4.00 kg, 8.86 mol, 1.00 equivalent) obtained in step 6 was dissolved in DMF (16.0 L), NaN₃ (650 g, 10.0 mol, 1.13 eq) was added at 25°C. The prepared mixture was stirred at 50°C for 10 hours in the presence of N₂. It was confirmed by TLC (petroleum ether/ethyl acetate = 3/1, Rf = 0.9) that the Chemical Formula 1G was consumed completely and one new spot was formed.

The reaction product obtained in 12 batches were combined and worked up. This mixture was poured into ice water (200 kg) while stirring. This was extracted with 2-MeTHF (100 L, 70 L, 50 L), and the combined organic layer was washed with brine (40 L x 2). The organic layer was used directly in the next step. Specifically, the crude compound 1H (crude, final theoretical amount: 42.4 kg) in 2-MeTHF (220 L) in the form of an orange solution was used in the next step without further purification.

### Step 8) Preparation of Compound 1-1

A solution in which the compound represented by Chemical Formula 1H (42.36 kg, 106 mol, 1.00 eq) obtained in step 7 was dissolved in 2-MeTHF (220 L) and H₂O (100 L), PPh₃ (27.9 kg, 106 mol, 1.20 eq) was added dividedly over 1 hour at 5°C in the presence of N₂. The prepared mixture was stirred at 25°C for 14 hours. It was confirmed by TLC (DCM/MeOH = 20/1, Rf = 0.02) that Chemical Formula 1H was consumed completely. NaCl (10 kg) and MTBE (200 L) were added to the prepared mixture, which was then isolated. The aqueous phase combined for the reaction was extracted with MTBE (200 L, 100 L). The combined organic layer was washed with brine (200 L), dried over Na₂SO₄, filtered, and concentrated to afford the residue. The residue was suspended in n-heptane (150 L), and stirred at 25°C for 30 minutes. The resulting mixture was filtered, and the filtrate was concentrated to afford a crude product. The crude product was dissolved in CH₃CN (300 L), and a solution in which benzoic acid (1.0 eq) was dissolved in CH₃CN (150 L) was added dropwise thereto at 5°C, and then the mixture was stirred at 15°C for 1 hour. The resulting solid was recrystallized using CH₃CN (350 L) at 70°C for 2 hours, and cooled to 15-25°C and filtered four times. The obtained solid was suspended in H₂O (100 L), and the pH value of the mixture was adjusted to 10 using NaOH (1 M). The mixture was extracted with MTBE (200 L, 100 L). The combined organic layer was washed with water (200 L) and brine (200 L), dried over Na₂SO₄, and then filtered and concentrated to afford Compound 1-1 as a yellow oil product (12.3 kg, 32.8 mol, yield: 30.9%, purity: 99.4%y).
¹H NMR (400 MHz CDCl₃): δ 4.06 (br s, 2H), 3.69 (br s, 1H), 2.65 - 2.82 (m, 3H), 2.20 (br s, 3H), 1.83-1.96 (m, 1H), 1.53 - 1.71 (m, 3H), 1.47-1.52 (m, 2H), 1.45 (s, 9H), 1.24 - 1.41 (m, 2H), 0.88 (s, 9H), 0.06 (s, 3H), 0.03 (s, 3H)

### Example 3: Preparation of tert-butyl (2R,3S)-3-((tert-butyldimethylsilyl)oxy)-2-(hydroxymethyl) piperidine-1-carboxylate

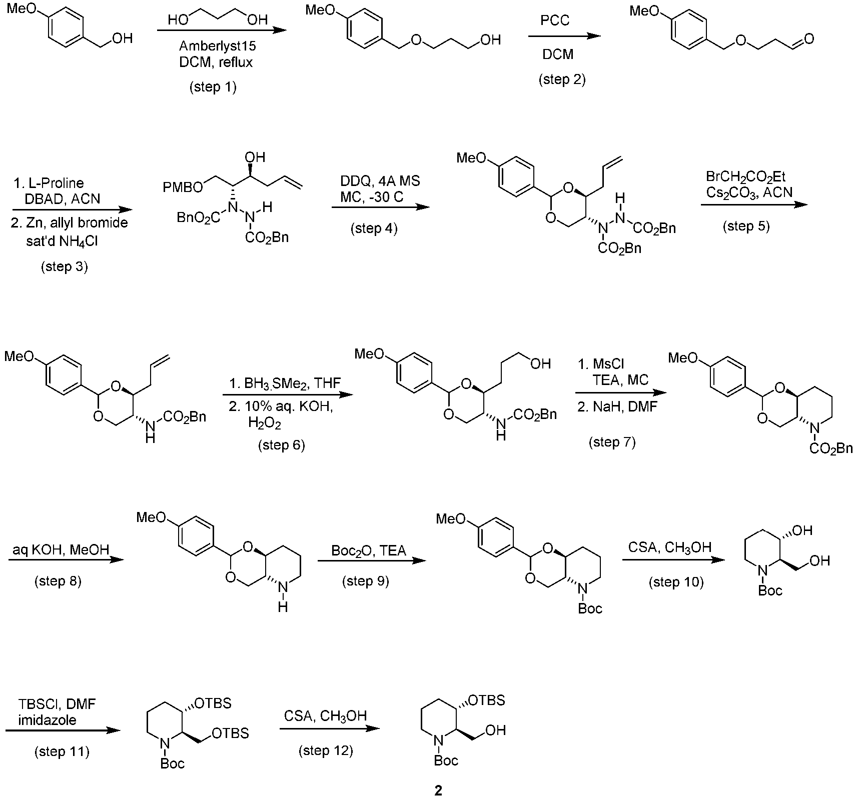

### Step 1) Preparation of 3-((4-methoxybenzyl)oxy)propan-1-ol

Starting material (4-methoxyphenyl)methanol (10 g), propane-1,3-diol (8 g, 1.5 eq) and Amberlyst^{®} 15 (1.5 g) were stirred at reflux in dichloromethane (DCM) (50 mL). After stirring overnight, it was confirmed that the starting material disappeared, and then the mixture was cooled to room temperature. After filtration, the solvent was removed, and purification by a silica column (EA:Hx=1:2) afforded the title compound 3-((4-methoxybenzyl)oxy)propan-1-ol as a colorless oil (13.4 g, 95%).
¹H NMR (400MHz, CDCl3) δ 1.85 (m, 2H), 2.38 (br s, 1H), 3.63 (t, 2H), 3.76 (m, 2H), 3.80 (s, 3H), 4.44 (s, 2H), 6.88 (d, 2H), 7.26 (d, 2H)

### Step 2) Preparation of 3-((4-methoxybenzyl)oxy)propanal

3-((4-Methoxybenzyl)oxy)propan-1-ol (13.4 g) obtained in step 1 was dissolved in DCM (200 mL). Celite (50 g) was added thereto. PCC (22.2 g, 1.5 eq) was added little by little under an ice bath, and then allowed to react at room temperature for about 4 hours. After filtration, the solvent was removed, and purification by a silica column (EA:Hx=1:4) afforded the title compound 3-((4-methoxybenzyl)oxy)propanal as a colorless oil (10 g, 74%).
¹H NMR (400MHz, CDCl3) δ 2.68 (t, 2H), 3.79 (m, 5H), 4.46 (s, 2H), 6.88 (d, 2H), 7.25 (d, 2H), 9.78 (s, 1H)

### Step 3) Preparation of dibenzyl 1-((2R,3S)-3-hydroxy-1-((4-methoxybenzyl)oxy)hex-5-en-2-yl)hydrazine-1,2-dicarboxylate

3-((4-Methoxybenzyl)oxy)propanal (1 g, 1.0 eq) obtained in step 2 was dissolved in ACN (15 mL). L-proline (593 mg, 1.0 eq) was added thereto. DBAD (986 mg, 1.1 eq) was added slowly at 0°C. The mixture was stirred at 0°C for 3 hours, and then sat'd NH₄Cl solution (3.5 mL) was added. Next, Zn (673 mg, 2.0 eq) and allyl bromide (0.873 mL, 2.0 eq) were added. The mixture was stirred at 0°C for 1 hour, EA and water were added to the reaction solution, and extracted. The organic layer was treated with MgSO₄ and filtered to remove the solvent. Purification by a silica column (EA:pet.ether=1:4) afforded the title compound, i.e., dibenzyl 1-((2R,3S)-3-hydroxy-1-((4-methoxybenzyl)oxy)hex-5-en-2-yl)hydrazine-1,2-dicarboxylate as a white solid (1.2 g, 57%).
¹H NMR (400MHz, CDCl3) δ 1.26 (t, 6H), 2.28 (br s, 2H), 3.50-3.76 (m, 3H), 3.81 (s, 3H), 3.96-4.24 (m, 5H), 4,46 (m, 2H), 5.13 (m, 2H), 5.85 (br s, 1H), 6.47 (m, 1H), 6.84 (d, 2H), 7.21 (d, 2H)

### Step 4) Preparation of dibenzyl 1-((4S,5R)-4-allyl-2-(4-methoxyphenyl)-1,3-dioxan-5-yl)hydrazine-1,2-dicarboxylate

4 Å molecular sieve (4 g) and DDQ (1.2 g, 1.1eq) were dispensed in DCM (25 mL), and then stirred for about 1 hour. Dibenzyl 1-((2R,3S)-3-hydroxy-1-((4-methoxybenzyl)oxy)hex-5-en-2-yl)hydrazine-1,2-dicarboxylate (2 g) obtained in step 3 was dissolved in DCM (20 mL) at 0°C, and added slowly thereto. When the starting material disappeared after stirring at 0°C for about 6 hours, sat'd sodium sulfate solution was added. After Celite filtration, the filtered solution was washed with sat'd NaHCO₃, water, and brine. The organic layer was treated with MgSO₄ and then filtered to remove the solvent. Purification by a silica column (EA:pet.ether=3:7) afforded the title compound, i.e., dibenzyl 1-((4S,5R)-4-allyl-2-(4-methoxyphenyl)-1,3-dioxan-5-yl)hydrazine-1,2-dicarboxylate (1.29 g, 65 %).
¹H NMR (400MHz, CDCl₃) δ 1.30 (t, 6H), 2.40 (br s, 1H), 2.60 (br s, 1H), 3.78 (s, 3H), 3.86-3.97 (m, 2H), 4.15-4.25 (m, 6H), 5.15 (m, 2H), 5.42 (s, 1H), 5.85 (m, 1H), 6.20 (m, 1H), 6.87 (d, 2H), 7.44 (d, 2H)

### Step 5) Preparation of benzyl ((4S,5R)-4-allyl-2-(4-methoxyphenyl)-1,3-dioxan-5-yl)carbamate

Dibenzyl 1-((4S,5R)-4-allyl-2-(4-methoxyphenyl)-1,3-dioxan-5-yl)hydrazine-1,2-dicarboxylate (6 g) obtained in step 4 was dissolved in dry ACN (110 mL). Cs₂CO₃ (14.36 g, 3.0 eq) and ethyl bromoacetate (2.44 mL, 1.5eq) were added thereto. The mixture was stirred at 50°C for 2 hours, and then stirred under reflux overnight. The reaction temperature was lowered to room temperature, and sat'd NH₄Cl solution was added to the reaction solution, and then extracted with EA. The organic layer was treated with MgSO₄ and then filtered to remove the solvent. Purification by a silica column (EA:pet.ether=1:3) afforded the title compound, i.e., benzyl ((4S,5R)-4-allyl-2-(4-methoxyphenyl)-1,3-dioxan-5-yl)carbamate as a white solid (3.4 g, 72%).
¹H NMR (400MHz, CDCl₃) δ 1.25 (t, 3H), 2.40-2.60 (m, 2H), 3.50 (m, 2H), 3.80 (s, 3H), 4.15 (m, 2H), 4.28-4.36 (m, 2H), 5.15 (m, 2H), 5.41 (s, 1H), 5.86 (m, 1H), 6.89 (d, 2H), 7.44 (d, 2H)

### Step 6) Preparation of benzyl ((4S,5R)-4-(3-hydroxypropyl)-2-(4-methoxyphenyl)-1,3-dioxan-5-yl)carbamate

Benzyl ((4S,5R)-4-allyl-2-(4-methoxyphenyl)-1,3-dioxan-5-yl)carbamate (1 g) obtained in step 5 was dissolved in dry THF (10 mL). BH₃·SMe₂ (2 M in THF, 1.85 mL 1.2 eq) was added slowly thereto at 0°C. The mixture was stirred overnight while slowly warming to room temperature. Water (2.5 mL), 3N NaOH (2.5 mL), and H₂O₂ (2.5 mL) were added thereto at 0°C, and stirred for about 2 hours. EA and brine were added to the reaction solution, and extracted. The organic layer was treated with MgSO₄ and filtered to remove the solvent. Purification by a silica column (EA:DCM=1:1) afforded the title compound, i.e., benzyl ((4S,5R)-4-(3-hydroxypropyl)-2-(4-methoxyphenyl)-1,3-dioxan-5-yl)carbamate (700 mg, 66%).
¹H NMR (400MHz, CDCl₃) δ 1.23 (t, 3H), 1.65-1.72 (m, 2H), 1.85-1.95 (m, 2H), 3.50-3.69 (m, 4H), 3.79 (s, 3H), 4.17 (m, 2H), 4.28 (m, 1H), 4.41 (m, 1H), 5.43 (s, 1H), 6.89 (d, 2H), 7.42 (d, 2H)

### Step 7) Preparation of benzyl (4αR,8αS)-2-(4-methoxyphenyl)hexahydro-5H-[1,3]dioxino[5,4-β]pyridine-5-carboxylate

Benzyl ((4S,5R)-4-allyl-2-(4-methoxyphenyl)-1,3-dioxan-5-yl)carbamate (700 mg) obtained in step 6 was dissolved in DCM (20 mL). TEA (0.58 mL, 2.0 eq) was added thereto. MsCl (0.16 mL 1.0 eq) was added slowly thereto at 0°C. When the starting material disappeared after stirring at 0°C for 2 hours, the reaction solution was quenched by adding sat'd NH₄Cl solution, and then extracted by adding water and DCM. The organic layer was washed once more with brine, treated with MgSO₄, and then filtered to remove the solvent. DMF (15 mL) was added to and dissolved in the crude compound. NaH (55% in mineral oil, 90 mg, 1.0 eq) was added slowly thereto at 0°C. When the starting material disappeared after stirring at 0°C for 2 hours, the reaction solution was quenched by adding sat'd NH₄Cl solution, and then extracted by adding water and EA. The organic layer was washed once more with brine, treated with MgSO₄, filtered to remove the solvent. Purification by a silica column (EA:pet. ether=4:6) afforded the title compound, i.e., benzyl (4αR,8αS)-2-(4-methoxyphenyl)hexahydro-5H-[1,3]dioxino[5,4-β]pyridine-5-carboxylate (600 mg, 90%).
¹H NMR (400MHz, CDCl₃) δ 1.23 (t, 3H), 1.60 (m, 2H), 1.79 (m, 1H), 2.15 (m, 1H), 2.79 (m, 1H), 3.21 (m, 1H), 3.68 (m, 1H), 3.80 (s, 3H), 4.18 (m, 3H), 4.42 (m, 1H), 4.79 (m, 1H), 5.47 (s, 1H), 6.89 (d, 2H), 7.42 (d, 2H)

### Step 8) Preparation of (4αR,8αS)-2-(4-methoxyphenyl)hexahydro-4H-[1,3]dioxino[5,4-β]pyridine

Benzyl (4αR,8αS)-2-(4-methoxyphenyl)hexahydro-5H-[1,3]dioxino[5,4-β]pyridine-5-carboxylate (600 mg) obtained in step 7 was dispersed in MeOH/H₂O (5 mL/5mL). KOH (5 g) was added thereto, and stirred under reflux for 5 hours. The reaction temperature was lowered to room temperature, MeOH was removed from the reaction solution, and EA and water were added and extracted. The organic layer was treated with MgSO₄ and then filtered to remove the solvent, thereby affording the title compound. i.e., (4αR,8αS)-2-(4-methoxyphenyl)hexahydro-4H-[1,3]dioxino[5,4-β]pyridine as a white solid (450 mg, 96%).
¹H NMR (400MHz, CDCl₃) δ 1.60 (m, 2H), 1.81 (m, 1H), 2.10 (m, 1H), 2.72 (m, 2H), 3.15 (m, 1H), 3.49 (m, 1H), 3.65 (m, 1H), 3.79 (s, 3H), 4.15 (m, 1H), 5.54 (s, 1H), 6.89 (d, 2H), 7.41 (d, 2H)
ESI mass [M+H]⁺: 250.2

### Step 9) Preparation of tert-butyl (4αR,8αS)-2-(4-methoxyphenyl)hexahydro-5H-[1,3]dioxino[5,4-β]pyridine-5-carboxylate

(4αR,8αS)-2-(4-methoxyphenyl)hexahydro-4H-[1,3]dioxino[5,4-β]pyridine (450 mg) obtained in step 8 was dissolved in DCM (20 mL). TEA (0.50 mL, 2.0 eq) and BocO (590 mg, 1.5 eq) were added under an ice bath, and stirred for about 2 hours. When the starting material disappeared, water and DCM were added to the reaction solution, and extracted. The organic layer was washed once more with brine, treated with MgSO₄, and filtered to remove the solvent. Purification by a silica column (EA:pet.ether=1:4) afforded the title compound, i.e., tert-butyl (4αR,8αS)-2-(4-methoxyphenyl)hexahydro-5H-[1,3]dioxino[5,4-β]pyridine-5-carboxylate (470 mg, 75%).
¹H NMR (400MHz, CDCl₃) δ 1.45 (s, 9H), 1.58 (m, 2H), 1.73 (m, 1H), 2.12 (m, 1H), 2.75 (m, 1H), 3.17 (m, 1H), 3.62 (m, 1H), 3.80 (s, 3H), 4.12 (m, 1H), 4.41 (t, 1H), 4.72 (m, 1H), 5.53 (s, 1H), 6.89 (d, 2H), 7.41 (d, 2H)

### Step 10) Preparation of tert-butyl (2R,3S)-3-hydroxy-2-(hydroxymethyl)piperidine-1-carboxylate

Tert-butyl (4αR,8αS)-2-(4-methoxyphenyl)hexahydro-5H-[1,3]dioxino[5,4-β]pyridine-5-carboxylate (470mg) obtained in step 9 was dissolved in MeOH (20 mL). CSA (156 mg, 0.5 eq) was added thereto under an ice bath, and stirred overnight. When the starting material disappeared, the solvent was removed. Purification by a silica column (EA) afforded the title compound, i.e., tert-butyl (2R,3S)-3-hydroxy-2-(hydroxymethyl)piperidine-1-carboxylate (180 mg, 58%).
¹H NMR (400MHz, CDCl₃) δ 1.43 (s, 9H), 1.71 (m, 2H), 1.82 (m,1H), 1.95 (m, 1H), 3.03 (m, 1H), 3.75 (m, 3H), 3.94 (m, 1H), 4.09 (m, 1H)

### Step 11) Preparation of tert-butyl (2R,3S)-3-((tert-butyldimethylsilyl)oxy)-2-(((tert-butyldimethylsilyl)oxy)methyl)piperidine-1-carboxylate

Tert-butyl (2R,3S)-3-hydroxy-2-(hydroxymethyl)piperidine-1-carboxylate (180 mg) obtained in step 10 was dissolved in DMF (3 mL). Imidazole (159 mg, 3.0 eq) and TBSCI (293 mg, 2.5 eq) were added under an ice bath, and stirred overnight. When the starting material disappeared, water and EA were added to the reaction solution, and extracted. The organic layer was washed once more with brine, treated with MgSO₄ and filtered to remove the solvent. Purification by a silica column (EA:pet.ether=1:9) afforded the title compound, i.e., tert-butyl (2R,3S)-3-((tert-butyldimethylsilyl)oxy)-2-(((tert-butyldimethylsilyl)oxy)methyl)piperidine-1-carboxylate (250 mg, 70%).
¹H NMR (400MHz, CDCl₃) δ 0.02 (m, 12H), 0.85 (m, 18H), 1.35 (m, 1H), 1.45 (s, 9H), 1.63 (m, 2H), 1.85 (m, 1H), 2.61 (m, 1H), 3.57 (m, 1H), 3.65 (t, 1H), 4.01 (m, 3H)

### Step 12) Preparation of tert-butyl (2R,3S)-3-((tert-butyldimethylsilyl)oxy)-2-(hydroxymethyl)piperidine-1-carboxylate

Tert-butyl (2R,3S)-3-((tert-butyldimethylsilyl)oxy)-2-(((tert-butyldimethylsilyl)oxy)methyl) piperidine-1-carboxylate (130 mg) obtained in step 11 was dissolved in MeOH (3 mL). CSA (33 mg, 0.5 eq) was added thereto under an ice bath, and stirred for about 2 hours. When the starting material disappeared, solid NaHCO₃ was added and quenched to remove the solvent. Purification by a silica column (EA:pet.ether=2:3) afforded the title compound, i.e., Compound 2, tert-butyl (2R,3S)-3-((tert-butyldimethylsilyl)oxy)-2-(hydroxymethyl)piperidine-1-carboxylate (95 mg, 99%).
¹H NMR (400MHz, CDCl₃) δ 0.05 (s, 3H), 0.09 (s, 3H), 0.85 (s, 9H), 1.38 (m, 1H), 1.45 (s, 9H), 1.59 (m, 2H), 1.85 (m, 1H), 2.81 (t, 1H), 3.62 (m, 2H), 3.86 (m, 1H), 3.96 (m, 1H), 4.17(m, 1H);
ESI mass [M+H]⁺: 368.3

### Comparative Example 2: Preparation of Compound 1-1 (tert-butyl (2R,3S)-2-(3-aminopropyl)-3-((tert-butyldimethylsilyl)oxy)piperidine-1-carboxylate)

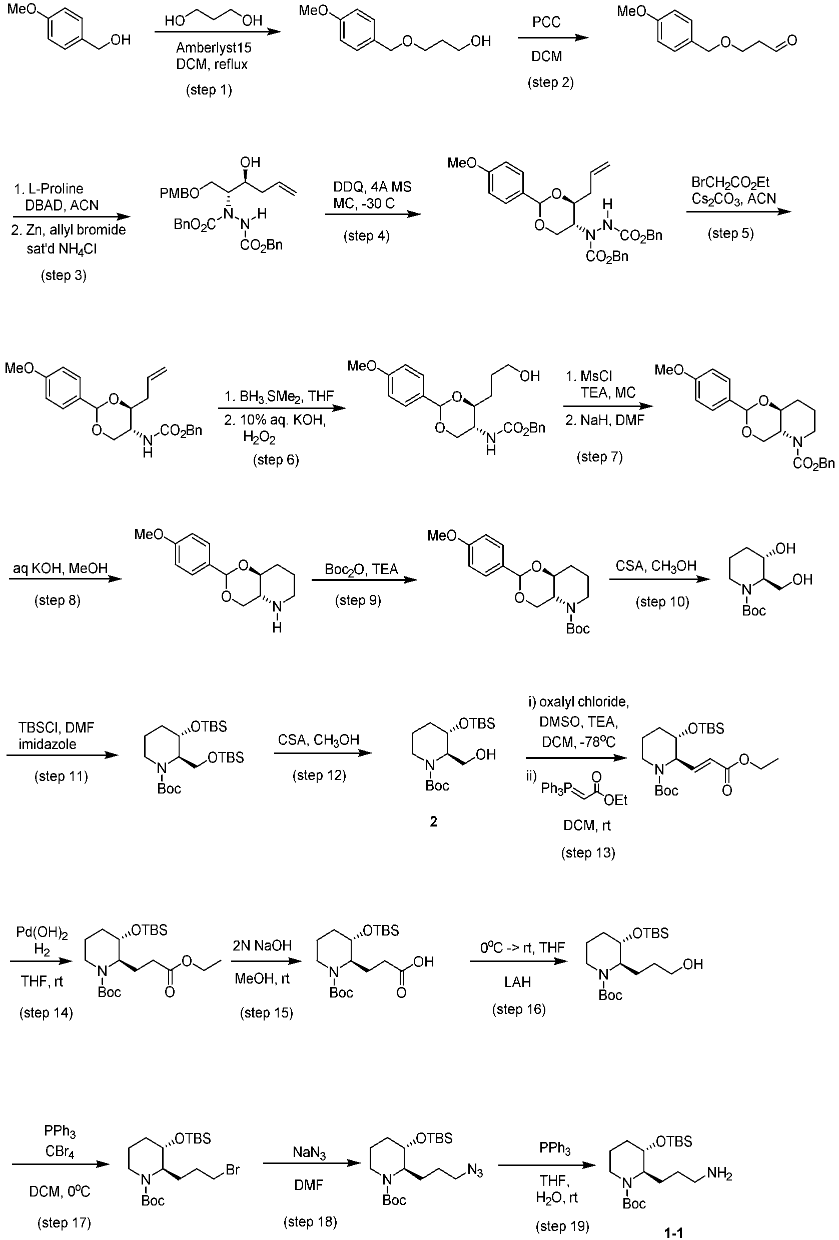

### Steps 1) to 12)

Steps 1) to 12) were carried out in the same manner as in Example 3 to afford Compound 2, i.e., tert-butyl (2R,3S)-3-((tert-butyldimethylsilyl)oxy)-2-(hydroxymethyl)piperidine-1-carboxylate.
¹H NMR (400MHz, CDCl₃) δ 0.05 (s, 3H), 0.09 (s, 3H), 0.85 (s, 9H), 1.38 (m, 1H), 1.45 (s, 9H), 1.59 (m, 2H), 1.85 (m, 1H), 2.81 (t, 1H), 3.62 (m, 2H), 3.86 (m, 1H), 3.96 (m, 1H), 4.17(m, 1H);
ESI mass [M+H]⁺: 368.3

### Step 13) Preparation of tert-butyl (2R,3S)-3-((tert-butyldimethylsilyl)oxy)-2-((E)-3-ethoxy-3-oxoprop-1-en-1-yl)piperidine-1-carboxylate

Dichloromethane (47 mL, 0.12 M) and oxalyl chloride (1.0 mL, 11.6 mmol) were placed in a flask filled with nitrogen, and then the reaction solution was lowered to -78°C. Then, N,N-dimethyl sulfoxide (1.7 mL, 23.2 mmol) was added thereto at the same temperature, and stirred for 30 minutes. Then, the Compound 2 obtained in step 12, i.e., tert-butyl (2R,3S)-3-((tert-butyldimethylsilyl)oxy)-2-(hydroxymethyl)piperidine-1-carboxylate (2.0 g, 5.8 mmol) was dissolved in a small amount of dichloromethane and added slowly thereto. After stirring at the same temperature for 1 hour, triethylamine (3.3 mL, 23.2 mmol) was added thereto, and the temperature of the reaction solution was raised from -78°C to room temperature. When the reaction was completed, the solvent was removed, diluted with ethyl acetate, and washed with a saturated sodium chloride solution. The organic layer was collected, dried with magnesium sulfate, filtered, and concentrated under reduced pressure. The concentrate was dissolved in dichloromethane (47 mL, 0.12 M), (carbetoxymethylene)triphenylphosphorane (4.0 g, 11.6 mmol) was added at room temperature, and stirred for 2 hours. When the reaction was completed, the solvent was removed, diluted with ethyl acetate, and washed with a saturated sodium chloride solution. The organic layer was collected, dried over magnesium sulfate, filtered, concentrated under reduced pressure, and then purified by column chromatography (hexane:ethyl acetate=4:1) to afford the title compound (2.1 g, yield 89%).

### Step 14) Preparation of tert-butyl (2R,3S)-3-((tert-butyldimethylsilyl)oxy)-2-(3-ethoxy-3-oxopropyl)piperidine-1-carboxylate

Tert-butyl (2R,3S)-3-((tert-butyldimethylsilyl)oxy)-2-((E)-3-ethoxy-3-oxoprop-1-en-1-yl)piperidine-1-carboxylate (3.2 g, 7.7 mmol) obtained in step 13 was dissolved in tetrahydrofuran (50 mL, 0.15 M), and palladium hydroxide (104 mg, 0.77 mmol) was added thereto. After connection a hydrogen balloon, the mixture was stirred at room temperature for 5 hours. When the reaction was completed, the reaction solution was filtered through Celite and concentrated under reduced pressure to afford the title compound. The resulting compound was used in the next reaction without purification.

### Step 15) Preparation of 3-((2R,3S)-1-(tert-butoxycarbonyl)-3-((tert-butyldimethylsilyl)oxy)piperidin-2-yl)propenoic acid

After tert-butyl (2R,3S)-3-((tert-butyldimethylsilyl)oxy)-2-(3-ethoxy-3-oxopropyl)piperidine-1-carboxylate ( 3.0 g, 7.2 mmol) obtained in step 14 was dissolved in methanol (20 mL, 0.36 M), 2N sodium hydroxide solution (10 mL) was added thereto, and stirred at room temperature for 3 hours. When the reaction was completed, the reaction mixture was neutralized with 1N aqueous hydrochloric acid, acidified, diluted with ethyl acetate, and washed with a saturated sodium chloride solution. The organic layer was collected, dried over magnesium sulfate, filtered, and concentrated under reduced pressure to afford the title compound. The resulting compound was used in the next reaction without purification.

### Step 16) Preparation of tert-butyl (2R,3S)-3-((tert-butyldimethylsilyl)oxy)-2-(3-hydroxypropyl)piperidine-1-carboxylate

After 3-((2R,3S)-1-(tert-butoxycarbonyl)-3-((tert-butyldimethylsilyl)oxy)piperidin-2-yl)propenoic acid (1.6 g, 4.0 mmol) obtained in step 15 was dissolved in tetrahydrofuran (50 mL, 0.08 M), the reaction solution was cooled to 0°C. Then, lithium aluminum hydride solution (1.6 mL, 4.0 mmol) was slowly added thereto, allowed to react at the same temperature for 30 minutes, and then stirred at room temperature for 2 hours. The reaction was completed by adding a small amount of water, diluted with ethyl acetate, and washed with a saturated sodium chloride solution. The organic layer was collected, dried with magnesium sulfate, filtered, concentrated under reduced pressure, and then purified by column chromatography (dichloromethane:methanol=10:1) to afford the title compound (1.3 g, yield: 85%).

### Step 17) Preparation of tert-butyl (2R,3S)-2-(3-bromopropyl)-3-((tert-butyldimethylsilyl)oxy)piperidine-1-carboxylate

Tert-butyl (2R,3S)-3-((tert-butyldimethylsilyl)oxy)-2-(3-hydroxypropyl)piperidine-1-carboxylate (5.1 g, 13.8 mmol) obtained in step 16 was added to dichloromethane (100 mL, 0.14 M). The reaction solution was cooled to 0°C, then triphenylphosphine (4.3 g, 16.5 mmol) and tetrabromomethane (5.5 g, 16.5 mmol) were added in that order at the same temperature, and stirred at room temperature for 2 hours. When the reaction was completed, the solvent was removed, diluted with ethyl acetate, and washed with a saturated sodium chloride solution. The organic layer was collected, dried over magnesium sulfate, filtered, concentrated under reduced pressure, and purified by a column chromatography (hexane:ethyl acetate=5:1) to afford the title compound (4.6 g, yield: 76%).

### Step 18) Preparation of tert-butyl (2R,3S)-2-(3-azidopropyl)-3-((tert-butyldimethylsilyl)oxy)piperidine-1-carboxylate

After tert-butyl (2R,3S)-2-(3-bromopropyl)-3-((tert-butyldimethylsilyl)oxy)piperidine-1-carboxylate (7.4 g, 17.0 mmol) obtained in step 17 was added to and dissolved in N,N-dimethylformamide (25 mL, 0.67 M), sodium azide (3.3 g, 17.0 mmol) was added thereto, and stirred at room temperature for 4 hours. When the reaction is completed, the solvent was removed, diluted with ethyl acetate, and washed with a saturated sodium chloride solution. The organic layer was collected, dried over sodium sulfate, filtered, concentrated under reduced pressure, and purified by a column chromatography (hexane:ethyl acetate=7:1) to afford the title compound (5.8 g, yield 85%).

### Step 19) Preparation of tert-butyl (2R,3S)-2-(3-aminopropyl)-3-((tert-butyldimethylsilyl)oxy)piperidine-1-carboxylate

After tert-butyl (2R,3S)-2-(3-azidopropyl)-3-((tert-butyldimethylsilyl)oxy)piperidine-1-carboxylate (5.5 g, 13.8 mmol) obtained in step 18 was dissolved in tetrahydrofuran (24 mL, 0.57 M), triphenylphosphine (4.3 g, 16.5 mmol) was added thereto, and stirred at room temperature for 30 minutes. Then, water (24 mL, 0.57 M) was added, and stirred at room temperature for 1 hour. When the reaction was completed, the reaction mixture was diluted with ethyl acetate and washed with a saturated sodium chloride solution. The organic layer was collected, dried over sodium sulfate, filtered, concentrated under reduced pressure, and purified by a column chromatography (dichloromethane:methanol=10:1 + triethylamine 2%) to afford the title compound, i.e., Compound 1-1 (4.0 g, yield: 78%).
¹H NMR (400 MHz CDCl₃): δ 4.06 (br s, 2H), 3.69 (br s, 1H), 2.65 - 2.82 (m, 3H), 2.20 (br s, 3H), 1.83-1.96 (m, 1H), 1.53 - 1.71 (m, 3H), 1.47-1.52 (m, 2H), 1.45 (s, 9H), 1.24 - 1.41 (m, 2H), 0.88 (s, 9H), 0.06 (s, 3H), 0.03 (s, 3H)

### Comparison of Example 2 and Comparative Example 2

In order to compare the case where Compound 1-1, the starting material of Example 1, was prepared by the preparation method of Example 2 and the case where it was prepared by that of Comparative Example 2, the yield of each preparation method was summarized in Table 2 below.

**[Table 2]**

| | Example 2 | Comparative Example 2 |
|---|---|---|
| Number of process step | 8 steps | 19 steps |
| | Step 1) 100% (in-situ) | Step 1) 95% |
| | Step 2) 66.41% | Step 2) 74% |
| | Step 3) 100% (in-situ) | Step 3) 57% |
| | Step 4) 52.8% | Step 4) 65% |
| | Step 5) 42.4% | Step 5) 72% |
| | Step 6) 100% (in-situ) | Step 6) 66% |
| | Step 7) 100% (in-situ) | Step 7) 90% |
| | Step 8) 30.9% | Step 8) 96% |
| | | Step 9) 75% |
| Yield of each step | | Step 10) 58% |
| | | Step 11) 70% |
| | | Step 12) 99% |
| | | Step 13) 89% |
| | | Step 14) 100% (in-situ) |
| | | Step 15) 100% (in-situ) |
| | | Step 16) 85% |
| | | Step 17) 76% |
| | | Step 18) 85% |
| | | Step 19) 78% |
| Final yield | 4.6% | 1.2% |

As shown in Table 2, it is confirmed that the preparation method of Example 2 can produce Compound 1-1 with improved yield while significantly shortening the process steps, as compared to the preparation method of Comparative Example 2.

Further, the preparation method of Example 2 shows that Compound 1-1 can be prepared on an industrial scale of 10 kg or more by minimizing column purification without using expensive reagents, unlike the preparation method of Comparative Example 2. Therefore, Compound 1-1 prepared according to the Example can be prepared and supplied in large quantities for the preparation of the compound represented by Chemical Formula 1, which is the final compound.

## Claims

1. A method for preparing a compound represented by the following Chemical Formula 1-1, the method comprising the steps of:
1) reacting a compound represented by the following Chemical Formula 1A with pyrrolidine to prepare a compound represented by the following Chemical Formula 1B;
2) reacting a compound represented by the following Chemical Formula 1B with 3-bromo-1-propene to prepare a compound represented by the following Chemical Formula 1C;
3) reducing a compound represented by the following Chemical Formula 1C in the presence of a ketoreductase enzyme to prepare a compound represented by the following Chemical Formula 1D;
4) reacting a compound represented by the following Chemical Formula 1D with a compound represented by the following Chemical Formula 1d to prepare a compound represented by the following Chemical Formula 1E;
5) ① reacting a compound represented by the following Chemical Formula 1E with a borane-based compound and then ② reacting the reactant with an oxidizing agent to prepare a compound represented by the following Chemical Formula 1F;
6) reacting a compound represented by the following Chemical Formula 1F with methanesulfonyl chloride in the presence of a base to prepare a compound represented by the following Chemical Formula 1G;
7) reacting a compound represented by the following Chemical Formula 1G with an azidation reagent to prepare a compound represented by the following Chemical Formula 1H; and
8) reacting a compound represented by the following Chemical Formula 1H in the presence of a base to prepare a compound represented by the following Chemical Formula 1-1: 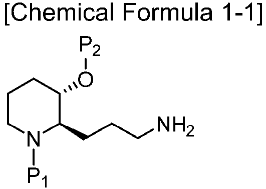 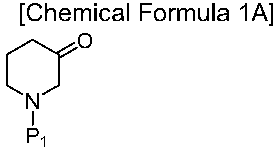 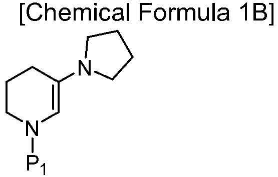 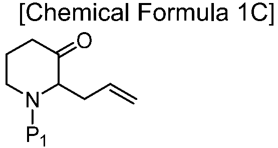 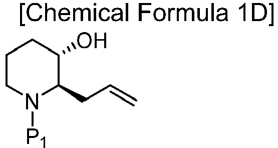
[Chemical Formula 1d] P₂-X 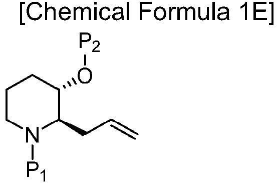 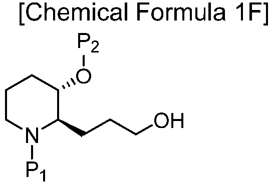 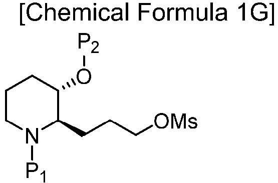 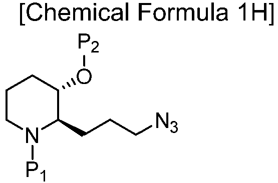
wherein, in Chemical Formulas 1-1, 1A to 1H and 1d,
X is halogen,
Ms is methanesulfonyl, and
P₁ and P₂ each independently represent a protecting group.

2. The preparation method according to claim 1, wherein:
in the step 1), the compound represented by Chemical Formula 1A and pyrrolidine is used in an equivalent ratio of 1:0.8 to 1:2.

3. The preparation method according to claim 1, wherein:
in the step 2), the compound represented by Chemical Formula 1B and 3-bromo-1-propene is used in an equivalent ratio of 1:0.7 to 1:2.

4. The preparation method according to claim 1, wherein:
in the step 3), the ketoreductase enzyme is dissolved and used in a buffer solution.

5. The preparation method according to claim 4, wherein:
the buffer solution is Na₂HPO₄ hydroxide, NaH₂PO₄ hydroxide, K₂HPO₄ hydroxide, or KH₂PO₄ hydroxide.

6. The preparation method according to claim 1, wherein:
in the step 3), the reduction is carried out in the presence of:
at least one enzyme selected from the group consisting of glutamate dehydrogenase (GDH) and glucose; and
at least one cofactor selected from the group consisting of nicotinamide adenine dinucleotide phosphate (NADP) and nicotinamide adenine dinucleotide (NAD).

7. The preparation method according to claim 1, wherein:
in the step 4), the compound represented by Chemical Formula 1D and the compound represented by Chemical Formula 1d is used in an equivalent ratio of 1:1 to 1:2.

8. The preparation method according to claim 1, wherein:
in the step 5), the compound represented by Chemical Formula 1E and the borane-based compound is used in an equivalent ratio of 1:0.5 to 1:1.0.

9. The preparation method according to claim 1, wherein:
in the step 5), the borane-based compound is borane dimethylsulfide (BH₃-Me₂S; BMS), or boranetetrahydrofuran.

10. The preparation method according to claim 1, wherein:
in the step 5), the oxidizing agent is at least one selected from the group consisting of hydrogen peroxide, Dess-Martin periodinane, and oxalyl chloride.

11. The preparation method according to claim 1, wherein:
in the step 6), the compound represented by Chemical Formula 1F and methanesulfonyl chloride is used in an equivalent ratio of 1:0.7 to 1:1.5.

12. The preparation method according to claim 1, wherein:
in the step 6), the base is at least one selected from the group consisting of triethylamine, diisopropyl ethylamine, pyridine, dimethylaniline, dimethylamino pyridine and sodium hydroxide.

13. The preparation method according to claim 1, wherein:
in the step 7), the azidation reagent is at least one selected from the group consisting of sodium azide, potassium azide and trimethylsilyl azide.

14. The preparation method according to claim 1, wherein:
in the step 7), the azidation reagent is used in an amount of 1 to 1.5 equivalents.

15. The preparation method according to claim 1, wherein:
in the step 8), the base is at least one selected from the group consisting of triphenyl phosphine, sodium borohydride, lithium aluminum hydride, and sodium hydride.

16. The preparation method according to claim 1, wherein:
the step 8) further comprises crystallizing the reaction product with acetonitrile.
